# EUROPÄISCHE PATENTSCHRIFT

(11) **EP 1 713 779 B1**
(45) Veröffentlichungstag und Bekanntmachung des Hinweises auf die Patenterteilung: **13.04.2011**
(21) Anmeldenummer: 05700954.0
(22) Anmeldetag: 15.01.2005
(51) Int. Cl.: C07D 231/56, A61K 31/416, A61P 3/10

(54) **INDAZOLDERIVATE ALS INHIBITOREN DER HORMON-SENSITIVEN LIPASE**
INDAZOLE DERIVATIVES AS INHIBITORS OF HORMONE-SENSITIVE LIPASES
DERIVES D'INDAZOLE UTILISES COMME INHIBITEURS DE LA LIPASE HORMONO-SENSIBLE

(30) Priorität: 02.02.2004 DE 102004005172
(43) Veröffentlichungstag der Anmeldung: 25.10.2006
(73) Patentinhaber: Sanofi-Aventis Deutschland GmbH, 65929 Frankfurt am Main (DE)
(72) Erfinder: ZOLLER, Gerhard, 61137 Schöneck (DE); PETRY, Stefan, 65779 Kelkheim (DE); MÜLLER, Günter, 65843 Sulzbach a. Ts. (DE); HEUER, Hubert, 55270 Schwabenheim (DE); BARINGHAUS, Karl-Heinz, 65926 Frankfurt am Main (DE)
(86) Internationale Anmeldenummer: PCT/EP2005/000365
(87) Internationale Veröffentlichungsnummer: WO 2005/073199

(56) Entgegenhaltungen:
- WO-A-03/035005
- WO-A-03/068773
- WO-A-03/097610
- WO-A-2004/022544
- WO-A-2004/046090
- WO-A-2004/062662
- DE-A1- 2 458 965
- LAN ET AL: "Structure-Activity Relationships of Pyrazole Derivatives as Cannabinoid Receptor Antagonists" JOURNAL OF MEDICINAL CHEMISTRY, AMERICAN CHEMICAL SOCIETY. WASHINGTON, US, Bd. 42, Nr. 4, 1999, Seiten 769-776, XP002145465 ISSN: 0022-2623

## Beschreibung

Die vorliegende Erfindung betrifft Indazolderivate der allgemeinen Formeln I oder II, deren pharmazeutisch anwendbare Salze und deren Verwendung als Arzneistoffe.

Pyrazolopyridinderivate mit hemmender Wiekung gegen GSK-3 und deren Verwendung in der Behandlung von Diabetes sind in WO 03/068773 beschrieben.

Indazolderivate zur Stimulierung des Cannabinoid Rezeptors sind in WO 03/035005 und Derivate der 3-Amino-Indazolcarbonsäure in DE 24 58 965 beschrieben. Ferner sind Phenylcarbamoyl-Indazole in WO 2004/046090 und 3-Amino-5-Phenylindazol-1-carbonsäureamid in US 2004/0097485 beschrieben, wobei diese beiden Dokumente erst nach Einreichung der vorliegenden Prioritätsanmeldung veröffentlicht wurden.

Gegenstand der Erfindung sind Indazolderivate der allgemeinen Formeln I oder II, 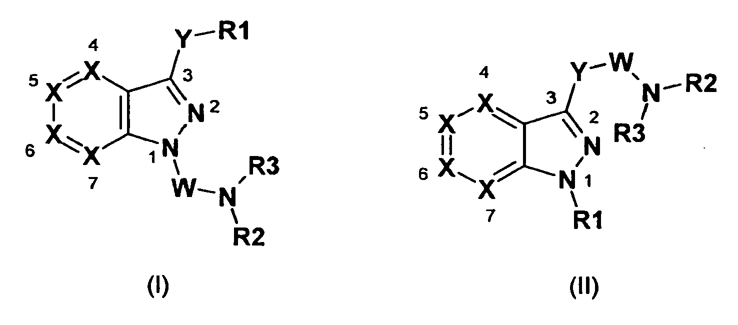 wobei bedeuten:
- W: -(C=O)-, -(S=O)-, -(SO₂)-
- X: =C(-R)- oder =N-;
- Y: -O-;
- R: Wasserstoff, Halogen, (C₁-C₆)-Alkyl, (C₁-C₃)-Alkyl-O-(C₁-C₃)-alkylen, Hydroxy, (C₁-C₆)-Alkylmercapto, Amino, (C₁-C₆)-Alkylamino, di-(C₂-C₁₂)- Alkylamino, mono-(C₁-C₆)-Alkylaminocarbonyl, di-(C₂-C₈)- Alkylaminocarbonyl, COOR4, Cyano, Trifluormethyl, (C₁-C₆)- Alkylsulfonyl, (C₁-C₆)-Alkylsulfinyl, Aminosulfonyl, Nitro, Pentafluorsulfanyl, (C₆-C₁₀)-Aryl, (C₅-C₁₂)-Heteroaryl, CO-NR2'R3', O- CO-NR2'R3', O-CO-(C₁-C₆)-Alkylen-CO-O-(C₁-C₆)-Alkyl, O-CO-(C₁-C₆)- Alkylen-CO-OH, O-CO-(C₁-C₆)-Alkylen-CO-NR2'R3' oder unsubstituiertes oder ein oder mehrfach durch F substituiertes (C₁-C₆)- Alkyloxy;
- R1: H, (C₁-C₆)-Alkyl, Benzyl;
- R2': H, (C₁-C₆)-Alkyl, (C₁-C₄)-Alkyl-Phenyl, (C₆-C₁₀)-Aryl, wobei Phenyl und Aryl ggf. durch Halogen, (C₁-C₆)-Alkyl, (C₁-C₃)-Alkyloxy, Hydroxy, (C₁- C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁- C₆)-Alkyloxycarbonyl, Cyano, Trifluormethyl, Trifluormethyloxy, (C₁-C₆)- Alkylsulfonyl, Aminosulfonyl, Nitro substituiert sein können oder Tetramethyl-tetrahydronaphthalin;
- R3': H, (C₁-C₆)-Alkyl; oder
- R2' und R3': zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR5-, -CR5R5-, -NR5-, -C(=O)-, -O-, -S-, -SO-, -SO₂-, ersetzt sein können, mit der Maßgabe, dass zwei Einheiten aus der Reihe -O-, -S-, -SO-, -SO₂- nicht benachbart sein dürfen;
- NR2R3: ein monocyclisches gesättigtes 5- bis 6-gliedriges Ringsystem, das in 4 Position ein Atom oder Atomglied aus der Reihe -CHR5-, -CR5R5-, -NR5-, -O-, -S-enthält;
- R4: Wasserstoff, (C₁-C₆)-Alkyl, Benzyl;
- R5: (C₁-C₆)-Alkyl, Halogen, Trifluormethyl, COOR4, Cyclopropyl, Cyclopropylen;
sowie deren physiologische verträglichen Salze und tautomeren Formen.

Bevorzugt sind Verbindungen der Formeln I und II, worin
- W: für -(C=O)- steht.

Ferner sind bevorzugt Verbindungen der Formeln I und II, worin
- X: in Position 4, 5 und 7 für =C(-R)- mit R = Wasserstoff steht.

Besonders bevorzugt sind Verbindungen der Formeln I oder II, worin
- W: -(C=O)-;
- X: =C(-R)- oder =N-;
- Y: -O-;
- R: Wasserstoff, Halogen, (C₁-C₆)-Alkyl, Hydroxy, Amino, COOR4, Trifluormethyl, (C₁-C₆)-Alkylsulfonyl, Nitro, Pentafluorsulfanyl, (C₆-C₁₀)-
- R1: Aryl, CO-NR2'R3', O-CO-NR2'R3' oder O-CO-(C₁-C₆)-Alkylen-CO-O- (C₁-C₆)-Alkyl; H, (C₁-C₆)-Alkyl, Benzyl;
- R2': (C₁-C₆)-Alkyl, Benzyl, (C₆-C₁₀)-Aryl oder Tetramethyl- tetrahydronaphthalin;
- R3': H, (C₁-C₆)-Alkyl; oder
- R2' und R3': zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis zwei Atome oder Atomgruppen aus der Reihe -CHR5-, -CR5R5-, -NR5-, -O-, -S-, ersetzt sein können, mit der Maßgabe, dass zwei Einheiten aus der Reihe -O-, -S- nicht benachbart sein dürfen;
- NR2R3: ein monocyclisches gesättigtes 5- bis 6-gliedriges Ringsystem, das in 4 Position ein Atom oder Atomglied aus der Reihe -CHR5-, -CR5R5-, -NR5-, -O-, -S- enthält;
- R4: Wasserstoff, (C₁-C₆)-Alkyl oder Benzyl bedeuten;
- R5: (C₁-C₆)-Alkyl, Halogen, Trifluormethyl, COOR4, Cyclopropyl, Cyclopropylen bedeuten.

Besonders bevorzugt sind ferner Verbindungen der Formel I, worin
- W: -(C=O)-;
- X: =C(-R)- oder =N-;
- Y: -O-;
- R: Wasserstoff, Halogen, Nitro, Hydroxy oder (C₁-C₆)-Alkyl;
- R1: H oder (C₁-C₆)-Alkyl;
- NR2R3: ein monocyclisches gesättigtes 5- bis 6-gliedriges Ringsystem, das in 4 Position ein Atom oder Atomglied aus der Reihe -CHR5-, -CR5R5-, -NR5-, -O-, -S- enthält;
- R5: (C₁-C₆)-Alkyl, oder Cyclopropyl bedeuten.

Besonders bevorzugt sind auch Verbindungen der Formel II, worin
- W: -(C=O)-;
- X: =C(-R)- oder =N-;
- Y: -O-;
- R: Wasserstoff, Halogen, (C₁-C₆)-Alkyl, Hydroxy, Amino, COOR4, Trifluormethyl, (C₁-C₆)-Alkylsulfonyl, Nitro, Pentafluorsulfanyl, (C₆-C₁₀)- Aryl, CO-NR2'R3', O-CO-NR2 'R3' oder O-CO-(C₁-C₆)-Alkylen-CO-O- (C₁-C₆)-Alkyl;
- R1: H, (C₁-C₆)-Alkyl oder Benzyl;
- R2': (C₁-C₆)-Alkyl, (C₆-C₁₀)-Aryl oder Tetramethyl-tetrahydronaphthalin;
- R3': H, (C₁-C₆)-Alkyl; oder
- R2' und R3': zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis zwei Atome oder Atomgruppen aus der Reihe -CHR5-, -CR5R5-, -NR5-, -O-, -S-, ersetzt sein können, mit der Maßgabe, dass zwei Einheiten aus der Reihe -O-, -S- nicht benachbart sein dürfen;
- NR2R3: ein monocyclisches gesättigtes 5- bis 6-gliedriges Ringsystem, das in 4 Position ein Atom oder Atomglied aus der Reihe -CHR5-, -CR5R5-, -NR5-, -O-, -S- enthält;
- R4: Wasserstoff, (C₁-C₆)-Alkyl, Benzyl ; und
- R5: (C₁-C₆)-Alkyl, Halogen, Trifluormethyl, COOR4, Cyclopropyl, Cyclopropylen bedeuten.

Ganz besonders bevorzugt sind die Verbindungen der Formel II, worin
- NR2R3: für Piperidin steht, das in 4 Position das Atomglied CHR5 enthält.

Die Erfindung bezieht sich auf Verbindungen der Formeln I oder II, in Form ihrer Salze, Racemate, racemischen Mischungen und reinen Enantiomere sowie auf ihre Diastereomere und Mischungen davon.

Die Alkylreste in den Substituenten R, R1, R2, R3, R4, R5 können sowohl geradkettig wie verzweigt sein. Halogen steht für Fluor, Chlor, Brom oder lod, besonders für Fluor oder Chlor.

Unter Aryl wird ein monocyclisches oder bicyclisches aromatischens Ring-System verstanden, das 6 bis 10 C-Atome im Ring oder in den Ringen enthält und das gegebenenfalls unabhängig voneinander durch ein bis vier Substituenten, bevorzugt ein oder zwei Substituenten - wie hierin beschreiben -substituiert sein kann.

Heteroaryl ist ein mono- oder bicyclisches aromatisches Ringsystem mit 5 bis 12 Ringgliedern, worin mindestens ein Atom im Ringsystem ein Heteroatom aus der Reihe N, O und S ist und die restlichen Atome C-Atome sind.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Der hier verwendete Begriff "physiologisch funktionelles Derivat" bezeichnet jedes physiologisch verträgliche Derivat einer erfindungsgemäßen Verbindung der Formel I oder II, z.B. einen Ester, der bei Verabreichung an einen Säuger, wie z.B. den Menschen, in der Lage ist, (direkt oder indirekt) eine Verbindung der Formel I oder II oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I oder II" auf Verbindung(en) der Formel I oder II, wie vorstehend beschrieben oder auf Verbindung(en) der Formel I oder II in denen die Reste folgende Bedeutungen aufweisen:
- W: -(C=O)-, -(S=O)-, -(SO₂)-;
- X: =C(-R)- oder =N-;
- Y: -O-;
- R: Wasserstoff, Halogen, (C₁-C₆)-Alkyl, (C₁-C₃)-Alkyl-O-(C₁-C₃)-alkylen, Hydroxy, (C₁-C₆)-Alkylmercapto, Amino, (C₁-C₆)-Alkylamino, di-(C₂-C₁₂)- Alkylamino, mono-(C₁-C₆)-Alkylaminocarbonyl, di-(C₂-C₈)- Alkylaminocarbonyl, COOR4, Cyano, Trifluormethyl, (C₁-C₆)- Alkylsulfonyl, (C₁-C₆)-Alkylsulfinyl, Aminosulfonyl, Nitro, Pentafluorsulfanyl, (C₆-C₁₀)-Aryl, (C₅-C₁₂)-Heteroaryl, CO-NR2'R3', O- CO-NR2'R3', O-CO-(C₁-C₆)-Alkylen-CO-O-(C₁-C₆)-Alkyl,O-CO-(C₁-C₆)- Alkylen-CO-OH, O-CO-(C₁-C₆)-Alkylen-CO-NR2'R3' oder unsubstituiertes oder ein oder mehrfach durch F substituiertes (C₁-C₆)- Alkyloxy;
- R1: H, (C₁-C₆)-Alkyl, Benzyl;
- R2, R2': H, (C₁-C₆)-Alkyl, (C₁-C₄)-Alkyl-Phenyl, (C₆-C₁₀)-Aryl, wobei Phenyl und Aryl ggf. durch Halogen, (C₁-C₆)-Alkyl, (C₁-C₃)-Alkyloxy, Hydroxy, (C₁- C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁- C₆)-Alkyloxycarbonyl, Cyano, Trifluormethyl, Trifluormethyloxy, (C₁-C₆)- Alkylsulfonyl, Aminosulfonyl, Nitro substituiert sein können oder Tetramethyl-tetrahydronaphthalin;
- R3, R3': H, (C₁-C₆)-Alkyl; oder
- R2 und R3 oder R2' und R3': zusammen mit dem sie tragenden Stickstoffatom ein monocyclisches, gesättigtes oder teilweise ungesättigtes 4- bis 7- gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR5-, -CR5R5-, -NR5-, - C(=O)-, -O-, -S-, -SO-, -SO₂-, ersetzt sein können, mit der Maßgabe, dass zwei Einheiten aus der Reihe -O-, -S-, -SO-, -SO₂- nicht benachbart sein dürfen;
- R4: Wasserstoff, (C₁-C₆)-Alkyl, Benzyl;
- R5: (C₁-C₆)-Alkyl, Halogen, Trifluormethyl, COOR4, Cyclopropyl, Cyclopropylen;
sowie deren physiologische verträglichen Salze und tautomeren Formen,
unter der Maßgabe, dass in den Verbindungen der Formel (I) mit W = CO
a) R2 und R3 zusammen mit dem sie tragenden N-Atom ein monocyclisches oder bicyclische Ringsystem bilden, falls Y = N(R1) mit R1 = H oder (C₁-C₆)-Alkyl oder
b) YR1, R2 und R3 nicht gleichzeitig nachfolgende Bedeutungen haben können:
   YR1 = OH, R2 = gegebenenfallls substituiertes (C₆-C₁₀)-Aryl und R3 = H;
sowie ihre Salze, Solvate und physiologisch funktionelle Derivate wie hierin beschrieben.

### Verwendung

Die erfindungsgemäßen Verbindungen der allgemeinen Formeln I oder II besitzen eine überraschende hemmende Wirkung an der Hormon Sensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.

Besonders geeignet sind solche Verbindungen zur Behandlung und/oder Prävention von
1. - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myökardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - lschämie/Reperfusions Syndrom
   - Akutes respiratorisches Distress Syndrom (ARDS) ("Schocklunge")
   - Lipodystrophie und lipodysstrophische Zustände, auch zur Behandlung von unerwünschten Wirkungen nach Arzneimitteln (z.B. Medikamente zur Behandlung von HIV oder Tumoren)

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I oder II selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I oder II abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I oder II enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I oder II mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I oder II, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I oder II mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I und II zeichnen sich durch günstige Wirkungen auf Stoffwechselstörungen aus. Sie beeinflussen den Fett- und Zuckerstoffwechsel positiv, sie senken insbesondere die Spiegel an FFA, Glycerol und Triglyceriden und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I oder II insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I oder II in Kombination mit Insulin verabreicht.

Bei einer Ausführungsform der Erfindung werden die Verbindungen der Formel I oder II in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel I oder II in Kombination mit einem Sulphonylharnstoff, wie z.B. Tolbutamid, Glibenclamid, Glipizid oder Glimepirid verabreicht.

Bei einer Ausführungsform werden die Verbindungen der Formel I oder II in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I oder II in Kombination mit einem Biguanid, wie z.B. Metformin, verabreicht.

Bei wieder einer Ausführungsform werden die Verbindungen der Formel I oder II in Kombination mit einem Meglitinid, wie z.B. Repaglinid, verabreicht.

Bei einer Ausführungsform werden die Verbindungen der Formel I oder II in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I oder II in Kombination mit einem DPPIV Inhibitor, wie z.B. in WO98/19998 WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel Ioder II in Kombination mit einem PPARgamma Agonist, wie z.B. Rosiglitazon, Pioglitazon, verabreicht.

Bei einer Aufführungsform werden die Verbindungen der Formel I oder II in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I oder II in Kombination mit einem α-Glukosidase-Inhibitor, wie z.B. Miglitol oder Acarbose, verabreicht.

Bei einer Ausführungsform werden die Verbindungen der Formel I oder II in Kombination mit mehr als einer der vorstehend genannten Verbindungen, z.B. in Kombination mit einem Sulphonylharnstoff und Metformin, einem Sulphonylharnsfoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I oder II in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht. Bei einer Ausführungsform der Erfindung werden die Verbindungen der Formel I oder II in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I oder II in Kombination mit einem polymeren Gallensäureadsorber, wie z.B. Cholestyramin, Colesevelam, verabreicht.

Bei einer Ausführungsform der Erfindung werden die Verbindungen der Formel I oder II in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I oder II in Kombination mit einem LDL-Rezeptorinduktor (siehe z.B. US 6,342,512) verabreicht.

Bei einer Ausführungsform werden die Verbindungen der Formel I oder II in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I oder II in Kombination mit einem PPARalpha Agonist verabreicht.

Bei einer Ausführungsform der Erfindung werden die Verbindungen der Formel I oder II in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I oder II in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I oder II in Kombination mit Nicotinsäure bzw. Niacin verabreicht.

Bei einer Ausführungsform der Erfindung werden die Verbindungen der Formel I oder II in Kombination mit einem CETP-Inhibitor, z.B. CP- 529, 414 (Torcetrapib), verabreicht.

Bei einer Ausführungsform der Erfindung werden die Verbindungen der Formel I oder II in Kombination mit einem ACAT-Inhibitor verabreicht

Bei einer Ausführungsform der Erfindung werden die Verbindungen der Formel loder II in Kombination mit einem MTP-Inhibitor, wie z.B. Implitapide, verabreicht.

Bei einer Ausführungsform der Erfindung werden die Verbindungen der Formel I oder II in Kombination mit einem Antioxidanz verabreicht.

Bei einer Ausführungsform der Erfindung werden die Verbindungen der Formel I oder II in Kombination mit einem Lipoprotein-Lipase Inhibitor, verabreicht.

Bei einer Ausführungsform der Erfindung werden die Verbindungen der Formel I oder II in Kombination mit einem ATP-Citrat-Lyase Inhibitor verabreicht.

Bei einer Ausführungsform der Erfindung werden die Verbindungen der Formel I oder II in Kombination mit einem Squalen Synthetase Inhibitor verabreicht.

Bei einer Ausführungsform der Erfindung werden die Verbindungen der Formel I oder II in Kombination mit einem Lipoprotein(a) Antagonist verabreicht.

### Antiobesita

Bei einer Ausführungsform der Erfindung werden die Verbindungen der Formel I oder II in Kombination mit einem Lipase Inhibitor, wie z.B. Orlistat, verabreicht.

Bei einer Ausführungsform ist der weitere Wirkstoff Fenfluramin oder Dexfenfluramin.

Bei noch einer Ausführungsform ist der weitere Wirkstoff Sibutramin.

Bei einer weiteren Ausführungsform werden die Verbindungen der Formel I oder II in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I oder II in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel oder II in Kombination mit anti-entzündlich wirkenden Medikamenten verabreicht.

Bei einer Ausführungsform werden die Verbindungen der Formel I oder II in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der erfindungsgemäßen Verbindungen der Formeln I oder II wurde an folgendem Enzymtestsytem geprüft:

### Substratpräparation:

### Präparation des NAG (NBD-Monoacylglycerid) Substrats:

6 mg Phosphatidylcholin und 6 mg Phosphatidylinositol werden in je 1 ml Chloroform gelöst. 10 mg NAG werden in 1 ml Chlorofom gelöst. Zwei Teile Phosphatidylinositollösung (z.B. 83.5 µl) und ein Teil Phosphatidylcholinlösung (z.B. 41.5 µl) und 100 µl NAG Lösung werden in Plastikszintillationsgefäßen zusammenpipettiert (Endkonzentration im Test: 0.0375 mg Phospholipid/ml; 0.05 mg/ NAG/ml). Das Chloroform (225 µl Gesamtvolumen) wird durch Überblasen mit einem N2-Strom komplett entfernt. Das getrocknete Substrat kann für bis zu 3 Tagen bei 4°C aufbewahrt werden. Zur Herstellung der Phospholipidvesikel/Micellen mit interkaliertem NAG (am Testtag) wird das getrocknete Substrat in 20 ml Assaypuffer (25 mM Tris/HCl, pH 7.4; 150 mM NaCl) aufgenommen und zwei Ultraschallbehandlungen mit einem Ultraschallstab (Branson Sonifier Type II, Standardmikrospitze): 1. Behandlung Einstellung 2, 2 x 1 min, dazwischen jeweils 1 min auf Eis; 2. Behandlung Einstellung 4, 2 x 1 min, dazwischen jeweils 1 min auf Eis. Während dieser Prozedur verändert sich sich die Farbe der Substratlösung von gelb (Extinktionsmaximum 481 nm) nach rot (Extinktionsmaximum 550 nm) durch Interkalation von NAG zwischen die Phospholipidmoleküle der Vesikel/Micellen. Vor Benutzung als Substrat (innerhalb der nächsten 2 h) wird die Lösung noch 15 min auf Eis inkubiert.

### Indirekter NAG Assay:

Der Assay wird in 1.5-ml Eppendorfgefäßen oder 96-Lochplatten für 60 min bei 30°C durchgeführt. Für das Auffinden von Inhibitoren der HSL werden 10 µl der Testsubstanz in Assaypuffer (25 mM Tris/HCl, pH 7.4; 150 mM NaCl) in Gegenwart von 16.6 % DMSO vorgelegt. 180 µl der Substratlösung (20 µg/ml Phosphatidylcholin, 10 µg/ml Phosphatidylinositol, 50 µg/ml NAG in Assaypuffer) werden hinzugegeben. Nach einer Vorinkubation für 15 min bei 30°C werden 20 µl der Enzymlösung in Assaypuffer (1- bis 4-fach verdünnt hinzupipettiert und die Extinktion bei 480 nm in einem Küvettenphotometer (0.5 ml-Küvette) bzw. Mikrotiterplattenlesegerät sofort gemessen. Nach 60 min Inkubation bei 30°C wird die Extinktion erneut gemessen. Die Zunahme der Extinktion bei 480 nm ist ein Maß für die Enzymaktivität. Unter Standardbedingungen führen 20 µg partiell gereinigte HSL zu einer Extinktionsänderung von 0.4 = 4000 arb. units.

### Direkter NAG Assay:

Alternativ zur Messung der Extinktionsänderung der Substratlösung werden die Produkte der HSL Reaktion durch Phasentrennung/Dünnschichtchromatographie untersucht. Dazu wird der Inkubationsansatz (200 µl Gesamtvolumen, s. indirekter NAG Assay) in 2 ml Eppendorfgefäßen mit 1.3 ml Methanol/Chloroform/Heptan (10:9:7) und anschließend mit 0.4 ml 0.1 M NaOH versetzt. Nach intensivem Mischen (10 sec) wird die Phasentrennung durch Zentrifugation (800xg, 20 min, Raumtemperatur) eingeleitet. Von der wässrigen oberen Phase werden äquivalente Volumen (z. B. 0.4 ml) abgenommen und die Extinktion photometrisch bei 481 nm bestimmt. Zur Dünnschichtchromatographie wird die wässrige Phase getrocknet (SpeedVac) und dann in 50 µl Tetrahydrofuran aufgenommen. 5-µl Proben werden auf Silicagel Si-60 Platten (Merck) aufgetragen. Die Chromatographie wird mit 78 ml Diethylether/22 ml Petroläther/1 ml Eisessig als Laufmittel durchgeführt. Die Menge an freigesetzter fluoreszierende NBD-Fettsäure wird durch Phosphorimaging (Molecular Dynamics, Storm 840 und ImageQuant Software) bei einer Anregungswellenlänge von 460 nm und Emissionswellenlänge von 540-560 nm bestimmt.

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 -407; G. Fredrikson et al., J. Biol. Chem. 256, 1981, 6311 - 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay:

Für die Herstellung des Substrats werden 25-50 µCi [3H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N2 getrocknet und dann in 2 ml 0.1 M KPi (pH 7.0) durch Ultraschallbehandlung (Branson 250, Mikrospitze, Einstellung 1-2, 2 x 1 min im 1-min Intervall) aufgenommen. Nach Zugabe von 1 ml KPi und erneuter Ultraschallbehandlung (4 x 30 sec auf Eis in 30-sec Intervallen) wird 1 ml 20% BSA (in KPi) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPi, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K2C03, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC50-Wertes erfolgt über eine Hemmkurve mit mind. 10 Konzentrationen der Testsubstanz. Für die Analyse der Daten wird das Softwarepaket GRAPHIT, Elsevier-BIOSOFT, benutzt.

In diesem Test zeigten die Verbindungen der Beispiele 1 bis 103

Inhibitionen im Bereich von IC₅₀ 1 nM - 1µM.

Die Herstellung der erfindungsgemäßen Verbindungen der allgemeinen Formeln I oder II erfolgt nach an und für sich bekannten Methoden z.B. durch Acylierung von substituierten, bzw. unsubstituiertem Indazolen III mit Carbamoylchloriden IV (Methode A), oder in zwei Stufen durch Umsetzung von Indazolen III mit Phosgen oder Äquivalenten wie Chlorcarbonsäuretrichlormethylester oder Carbonsäureditrichlormethylester und weiterer Umsetzung des erhaltenen Indazolcarbonsäurechlorids mit Aminen oder Anilinen (Methode B). Für Verbindungen bei denen R3 Wasserstoff ist, können die Indazole III auch mit den entsprechenden Isocyanaten R2-N=C=O umgesetzt werden.

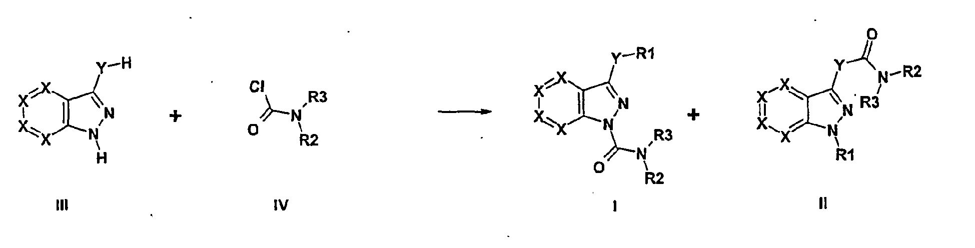

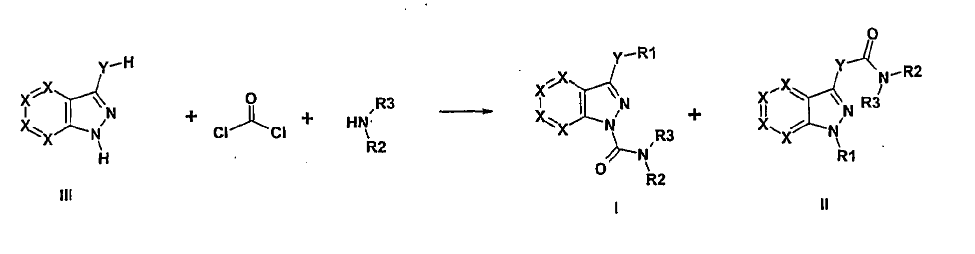

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.

Die als Ausgangsverbindungen III eingesetzten Indazole oder entsprechenden Azasubstituierten Derivate sind kommerziell erhältlich oder lassen sich nach Literatur bekannten Verfahren herstellen (z.B. L. Baiocchi, G. Corsi Synthesis (1978), 633-648, I. Sekikawa et al. J. Het. Chem. (1973), 931-932).

Die Verbindungen der allgemeinen Formeln I oder II werden nach allgemein bekannten Verfahren durch Chromatographie getrennt und gereinigt.

Die nachfolgend aufgeführten Beispiele dienen zur Erläuterung der Erfindung, ohne diese jedoch einzuschränken.

### Beispiele

### Beispiel 1:

### 4-Methyl-piperidine-1-carbonsäure 1H-indazol-3-yl ester

300 mg (2.24 mmol) 1H-Indazol-3-ol wurden in 25 ml THF gelöst und auf-20°C abgekühlt: Man tropfte 1.3 ml (2.46 mmol) Phosgen in Toluol (20 proz.) zu und rührte 90 min., wobei sich das Reaktionsgemisch auf Raumtemperatur erwärmte. Das Reaktionsgemisch wurde eingeengt und nochmals mit einigen ml Toluol evakuiert. Der Rückstand wurde in 15 ml THF gelöst und 265 µl (2.2 mmol) 4-Methyl-piperidin zugetropft, 3 h bei Raumtemperatur gerührt, eingeengt und über präparative HPLC (PR18, Acetonitril/Wasser 0.1 % TFA) gereinigt. Ausbeute: 347 mg (60%), M+H+: 260.4.

### Beispiel 2:

### 4-Methyl-piperidine-1-carbonyl chloride

9 g (90.75 mmol) 4-Methyl-piperidin und 13.9 ml (100 mol) Triethylamin wurden in 100 ml THF gelöst und bei - 30°C 54.9 ml (100 mmol) Phosgen in Toluol (20 proz.) zugegeben und 2.5 h gerührt, wobei sich das Reaktionsgemisch auf Raumtemperatur erwärmte. Das Reaktionsgemisch wurde eingeengt und der Rückstand mit Methylenchlorid versetzt, abfiltriert und das Filtrat eingeengt. Das Rohprodukt (12.7 g) wurde ohne weitere Reinigung umgesetzt.

### Beispiel 3:

### 4-Methyl-piperidine-1-carbonsäure 4-fluoro-1H-indazol-3-yl ester

100 mg (0.66 mmol) 4-Fluoro-1H-indazol-3-ol und 116.8 mg (0.72 mmol) 4-Methyl-piperidine-1-carbonyl chloride (Beispiel 2) wurden in 10 ml Pyridin 4 h unter Rückfluss erhitzt und über Nacht stehen gelassen. Nach Zugabe von 24 mg 4-Methyl-piperidine-1-carbonyl chloride wurde nochmals 2 h unter Rückfluss erhitzt, das Pyridin im Vakuum abdestilliert, der Rückstand in Wasser gelöst und mit Ethylacetat extrahiert. Die organische Phase wurde eingeengt und über präparative HPLC (PR18, Acetonitril/Wasser 0.1% TFA) gereinigt. Ausbeute: 56 mg (31 %), M+H+: 278.1.

### Beispiel 4:

### 4-Methyl-piperidine-1-carbonsäure 1-methyl-1H-indazol-3-yl ester

80.1 mg (0.31 mmol) 4-Methyl-piperidine-1-carbonsäure 1H-indazol-3-yl ester (Beispiel 1), 38.1 mg (0.34 mmol) Kalium-tert-butylat und 48.2 mg (0.34 mmol) lodmethan wurden 48 h bei Raumtemperatur gerührt. Das Lösungsmittel wurde im Vakuum abdestilliert, der Rückstand in Wasser gelöst und mit Ethylacetat extrahiert. Die organische Phase wurde eingeengt und über präparative HPLC (PR18, Acetonitril/Wasser 0.1 % TFA) gereinigt. Ausbeute: 7 mg (8%), M+H+: 274.1.

### Beispiel 5:

100 mg (0.56 mmol) 6-Nitro-1H-indazol-3-ol und 135.3 mg (0.83 mmol) 4-Methyl-piperidine-1-carbonyl chloride (Beispiel 2) wurden in 10 ml Pyridin 5 h unter Rückfluss erhitzt und über Nacht stehen gelassen. Pyridin wurde im Vakuum abdestilliert, der Rückstand in Wasser gelöst und mit Ethylacetat extrahiert. Die organische Phase wurde eingeengt und über präparative HPLC (PR18, Acetonitril/Wasser 0.1% TFA) gereinigt. Ausbeute: 5 mg (3%) A: (3-Hydroxy-6-nitro-indazol-1-yl)-(4-methyl-piperidin-1-yl)-methanone, M+H+: 305.1 und 64 mg (38%) B: 4-Methyl-piperidine-1-carbonsäure 6-nitro-1H-indazol-3-yl ester, M+H+: 305.1.

### Beispiel 6:

### 4-Methyl-piperidine-1- carbonsäure 5-nitro-1H-indazol-3-yl ester

200 mg (1.12 mmol) 5-Nitro-1H-indazol-3-ol und 180.4 mg (1.67 mmol) 4-Methyl-piperidine-1-carbonyl chloride (Beispiel 2) wurden in 20 ml Pyridin 5 h unter Rückfluss erhitzt und über Nacht stehen gelassen. Pyridin wurde im Vakuum abdestilliert, der Rückstand in Wasser gelöst und mit Ethylacetat extrahiert. Die organische Phase wurde eingeengt und über präparative HPLC (PR18, Acetonitril/Wasser 0.1 % TFA) gereinigt. Ausbeute: 48 mg (14%), M+H+: 304.99.

### Beispiel 7:

### 4-Methyl-piperidine-1-carbonsäure 6-amino-1H-indazol-3-yl ester

30 mg (0.1 mmol) 4-Methyl-piperidine-1-carbonsäure 6-nitro-1H-indazol-3-yl ester (Beispiel 5B) wurden in 15 ml Ethanol in Gegenwart von 10% Palladium/Kohle bei 2 bar Wasserstoffdruck 2.5 h bei Raumtemperatur hydriert. Der Katalysator wurde abgesaugt und das Filtrat eingeengt. Ausbeute: 21 mg (76%), M+H+: 275.2.

### Beispiel 8:

Beispiel 1 wurde mit 2 g (14.9 mmol) 1H-Indazol-3-ol wiederholt. Dabei konnten neben A: 4-Methyl-piperidine-1-carbonsäure 1H-indazol-3-yl ester auch noch die Isomeren Produkte B: (3-Hydroxy-indazol-1-yl)-(4-methyl-piperidin-1-yl)-methanone und C: 2-(4-Methyl-piperidine-1-carbonyl)-1,2-dihydro-indazol-3-one isoliert werden.

### Beispiel 9:

### 4-Methyl-piperidine-1-carbonsäure 1H-pyrazolo[3,4-b]pyridin-3-yl ester

a) 1H-Pyrazolo[3,4-b]pyridin-3-ol: 5 g (26.94 mmol) 2-Chloro-Nicotinsäure ethyl ester und 4.76 g (80.82 mmol) Hydrazin Hydrat (85 proz.) wurden in 10 ml Ethanol 6 h unter Rückfluss erhitzt. Das Reaktionsgemisch wurde eingeengt. Ausbeute: 3.5g (96%), M+H+: 135.9.
b) 300 mg (2.2 mmol) 1H-Pyrazolo[3,4-b]pyridin-3-ol und 538.2 mg (3.3 mmol) 4-Methyl-piperidine-1-carbonyl chloride (Beispiel 2) wurden in 25 ml Pyridin 4 h unter Rückfluss erhitzt und über Nacht stehen gelassen. Nach Zugabe von 24 mg 4-ethyl-piperidine-1-carbonyl chloride wurde nochmals 2 h unter Rückfluss erhitzt, das Pyridin im Vakuum abdestilliert, der Rückstand in Wasser gelöst und mit Ethylacetat extrahiert. Die organische Phase wurde eingeengt und über präparative HPLC (PR18, Acetonitril/Wasser 0.1 % TFA) gereinigt. Ausbeute: 99 mg (17%), M+H+: 261.28.

### Beispiel 10:

500 mg (2.16 mmol) 1H-Indazol-3-ylamine; compound with sulfuric acid und 419.3 mg (2.59 mmol) 4-Methyl-piperidine-1-carbonyl chloride (Beispiel 2) und 300 µl (4.32 mmol) Triethylamin wurden in 30 ml Pyridin 5 h unter Rückfluss erhitzt und über Nacht stehen gelassen. Nach Zugabe von 302 µl Triethylamin und und 390 mg 4-Methyl-piperidine-1-carbonyl chloride wurde nochmals 2.5 h erhitzt. Pyridin wurde im Vakuum abdestilliert, der Rückstand in Wasser gelöst und mit Ethylacetat extrahiert. Die organische Phase wurde eingeengt und über präparative HPLC (PR18, Acetonitril/Wasser 0.1 % TFA) gereinigt. Ausbeute: 55 mg (10%) A: 4-Methyl-piperidine-1-carbonsäure (1H-indazol-3-yl)-amide, M+H+: 259.1 und 36.4 mg (7%) B: (3-Amino-indazol-1-yl)-(4-methyl-piperidin-1-yl)-methanone, M+H+: 259.1.

### Beispiel 11:

### 4-Trifluoromethyl-piperidine-1-carbonyl chloride

Carbonic acid ditrichloromethyl ester (840 mg, 2.83 mmol) wurden in 30 ml Methylenchlorid gelöst und im Eisbad langsam mit 2.06 ml (25.24 mmol) Pyridin versetzt. Nach 30 min. wurden 4-Trifluoromethyl-piperidin-hydrochlorid (1.45 g, 7.65 mmol) langsam portionsweise zugegeben. Nach Entfernen des Eisbades wurde noch 90 min. nachgerührt, der Niederschlag abgesaugt, mit n-Heptan nachgewaschen und das Filtrat eingeengt. Das erhaltene Produkt (1.9 g) enthält noch etwas Salz und wurde direkt weiter umgesetzt.

### Beispiel 12:

### 4-Trifluoromethyl-piperidine-1-carboxylic acid 6-hydroxy-4-methyl-1H-pyrazolo[3,4-b]pyridin-3-yl ester

4-Methyl-1H-pyrazolo[3,4-b]pyridine-3,6-diol (1 g, 6.05 mmol), 4-Trifluoromethyl-piperidine-1-carbonyl chloride (1.436 g, 6.6 mmol) und Triethylamin (1.68 ml, 12.11 mmol) wurden in 25 ml Pyridin 1 h bei Raumtemp. gerührt. Nach Zugabe von 0.5 ml Triethylamin wurde 2 h weitergerührt, eingengt und mit Ethylacetate und Wasser versetzt. Der erhaltene Niederschlag wurde abgesaugt und getrocknet. Ausbeute: 765 mg (37%) 4-Trifluoromethyl-piperidine-1-carboxylic acid 6-hydroxy-4-methyl-1H-pyrazolo[3,4- b]pyridin-3-yl ester. Die organische Phase wurde abgetrennt, eingeengt und und über präparative HPLC (PR18, Acetonitril/Wasser 0.1 % TFA) gereinigt. Ausbeute: 96 mg (5 %) 4-Trifluoromethyl-piperidine-1-carboxylic acid 6-hydroxy-4-methyl-1H-pyrazolo[3,4- b]pyridin-3-yl ester, M+H+: 345.13; 102 mg (3%) 4-Trifluoromethyl-piperidine-1-carboxylic acid 4-methyl-3-(4-trifluoromethyl-piperidine-1-carbonyloxy)-1H-pyrazolo[3,4- b]pyridin-6-yl ester, M+H+: 524.20; 106 mg (3%) 4-Trifluoromethyl-piperidine-1-carboxylic acid 6-hydroxy-4-methyl-1-(4-trifluoromethylpiperidine-1-carbonyl)-1H-pyrazolo[3,4-b]pyridin-3-yl ester, M+H+: 524.52; 54 mg (1.3%) 4-Trifluoromethyl-piperidine-1-carboxylic acid 4-methyl-3-(4-trifluoromethylpiperidine-1-carbonyloxy)-1-(4-trifluoromethyl-piperidine-1-carbonyl)-1H-pyrazolo[3,4-b]pyridin-6-yl ester, M+H+: 703.36.

### Beispiel 13:

### 4-Methyl-piperazine-1-carboxylic acid 4-methyl-3-(4-trifluoromethyl-piperidine-1-carbonyloxy)-1H-pyrazolo[3,4-b]pyridin-6-yl ester; compound with trifluoro-acetic acid

4-Trifluoromethyl-piperidine-1-carboxylic acid 6-hydroxy-4-methyl-1H-pyrazolo[3,4-b]pyridin-3-yl ester (300 mg, 0.87 mmol), 4-Methyl-piperazine-1-carbonyl chloride hydrochlorid (191 mg, 0.96 mmol) und Triethylamin (0.48 ml, 3.48 mmol) wurden in 10 ml Pyridin 5 h bei Raumtemp. gerührt. Nach Zugabe von 0.4 ml Triethylamin und 100 mg 4-Methyl-piperazine-1-carbonyl chloride hydrochlorid wurde 1 h weitergerührt, eingengt und mit Ethylacetate und Wasser versetzt und auf pH 8 gestellt. Die organische Phase wurde abgetrennt (mehrmalige Extraktion), eingeengt und über präparative HPLC (PR18, Acetonitril/Wasser 0.1% TFA) gereinigt. Ausbeute: 125 mg (25 %) 4-Methyl-piperazine-1-carboxylic acid 4-methyl-3-(4-trifluoromethyl-piperidine-1-carbonyloxy)-1H-pyrazolo[3,4-b]pyridin-6-yl ester; compound with trifluoro-acetic acid, M+H+: 471.24; 82 mg (13%) 4-Trifluoromethyl-piperidine-1-carboxylic acid 6-hydroxy-4-methyl-1-(4-methyl-piperazine- 1-carbonyl)-1H-pyrazolo[3,4-b]pyridin-3-yl ester; compound with trifluoro-acetic acid, M+H+: 471.27.

### Beispiel14:

### 6-Nitro-1H-indazol-3-ol

2-Fluoro-4-nitro-benzoic acid methyl ester (5 g, 25.11 mmol) und Hydrzinhydrat (1.34 ml, 27.62 mmol) wurden in 250 ml Ethanol gelöst und 11 h unter Rückfluß erhitzt. Es wurden nochmals 0.26 ml Hydrzinhydrat zugegeben und weitere 6 H unter Rückfluß erhitzt, eingeengt und mit Ethylacetat und Wasser versetzt. Der ausgefallene Rückstand wurde abgesaugt und getrocknet. Nach Reinigung über präparative HPLC (PR18, Acetonitril/Wasser 0.1 % TFA) erhielt man 1.39 g Produkt, M+H+: 180.05.

### Beispiel15:

### 6-Fluoro-1H-indazol-3-ol:

2-Amino-4-fluoro-benzoic acid (25 g, 161.2 mmol) wurden 250 ml Wasser und 39 ml konz. Salzsäure suspendiert. Bei 0°C wurden Natriumnitrit (11.2 g, 161.2 mmol) in 30 ml Wasser unterhalb 10°C zugetropft. Nach 30 min.,bei Raumtemp. wurden Natriumsulfit (69 g, 400 mmol) in 250 ml Wasser zugegeben. Nach 2 h Rühren wurden 30 ml konz. Salzsäure zugegeben und über Nacht stehen gelassen. Dann wurde 9 h unter Rückfluß erhitzt, abgekühlt und mit Natrimhydrogencarbonat auf pH 5.5 eingestellt. Der Niederschlag wurde abgesaugt und getrocknet. Ausbeute: 19.8 g, (81%),M+H+: 152.94.

### Beispiel16:

### 3-Hydroxy-1H-indazole-6-carboxylic acid

2-Amino-terephthalic acid dimethyl ester (5 g, 23.9 mmol) wurden in 40 ml Wasser und 6 ml konz. Salzsäure Salzsäure gelöst. Bei 0°C wurden Natriumnitrit (1.65 g, 23.9 mmol) in 5 ml Wasser unterhalb 10°C zugetropft. Nach 30 min. bei Raumtemp. wurden Natriumsulfit (11.02 g, 87.42 mmol) in 40 ml Wasser zugegeben. Nach 1 h Rühren wurden 10 ml konz. Salzsäure zugegeben und über Nacht stehen gelassen. Dann wurde 24 h auf 80°C erhitzt, abgekühlt und mit Natronlauge auf pH 5.5 eingestellt. Der Niederschlag wurde abgesaugt und getrocknet. Ausbeute: 2.29 g, (54%), M+H+: 179.04.

### Beispiel17:

### 3-Hydroxy-4-methyl-1H-pyrazolo[3,4-b]pyridine-6-carboxylic acid

5-Amino-2H-pyrazol-3-ol (3.1 g, 31.6 mmol) wurden in 100 ml Methanol suspendiert. Dann wurden Natriummethylat (5.1 g, 95 mmol) und 2,4-Dioxo-pentanoic acid ethyl ester (5 g, 31.6 mmol) zugegeben und 12 h unter Rückfluß erhitzt. Das Lösungsmittel wurde abrotiert und der Rückstand mit Wasser versetzt, der Niederschlag abgesaugt und das Filtrat mit verdünnter salzsäure auf pH 6 gestellt. Das ausgefallene Produkt (beide Niederschläge) wurde abgesaugt und getrocknet. Ausbeute: 4.9 g (70%), M+H+: 194.09.

Analog der beschriebenen Beispiele wurden folgende Verbindungen hergestellt:

| Beispiel | Name | M+H+ |
|---|---|---|
| 18 | 3-Methyl-piperidine-1-carboxylic acid 1H-indazol-3-yl ester | 260.2 |
| 19 | (3,4-Dihydro-2H-quinolin-1-yl)-(3-hydroxy-indazol-1-yl)-methanone | 294.3 |
| 20 | (3-Hydroxy-indazol-1-yl)-pyrrolidin-1-yl-methanone | 232.3 |
| 21 | (3-Hydroxy-indazol-1-yl)-thiomorpholin-4-yl-methanone | 264.4 |
| 22 | (3,4-Dihydro-1H-isoquinolin-2-yl)-(3-hydroxy-indazol-1-yl)-methanone | 294.5 |
| 23 | 3-Hydroxy-indäzole-1-carboxylic acid methyl-phenyl-amide | 268.3 |
| 24 | 3-Methyl-piperidine-1-carboxylic acid 1-methyl-1H-indazol-3-yl ester | 274.4 |
| 25 | (3-Methoxy-indazol-1-yl)-(3-methyl-piperidin-1-yl)-methanone | 274.4 |
| 26 | 3-Hydroxy-indazole-1-carboxylic acid dibutylamide | 290.16 |
| 27 | Diethyl-carbamic acid 1H-indazol-3-yl ester | 234.11 |
| 28 | Diisopropyl-carbamic acid 1H-indazol-3-yl ester | 262.13 |
| 29 | Piperidine-1-carboxylic acid 1H-indazol-3-yl ester | 246.10 |
| 30 | (1,3-Dihydro-isoindol-2-yl)-(3-hydroxy-indazo!-1-yl)-methanone | 280.12 |
| 31 | 4-Methyl-piperidine-1-carboxylic acid 6-trifluoromethyl-1H-indazol-3-yl ester | 328.16 |
| 32 | 4-Methyl-piperidine-1-carboxylic acid 6-fluoro-1H-indazol-3-yl ester | 278.13 |
| 33 | 4-Methyl-piperidine-1-carboxylic acid 6-chloro-1H-indazol-3-yl ester | 294.06 |
| 34 | 4-Methyl-piperidine-1-carboxylic acid 6-methyl-1H-indazol-3-yl ester | 274.12 |
| 35 | (6-Chloro-3-hydroxy-indazol-1-yl)-(4-methyl-piperidin-1-yl)-methanone | 294.12 |
| 36 | (3-Hydroxy-indazol-1-yl)-(octahydro-isoindol-2-yl)-methanone | 286.16 |
| 37 | Octahydro-isoindole-2-carboxylic acid 1H-indazol-3-yl ester | 286.17 |
| 38 | 3,4-Dihydro-1H-isoquinoline-2-carboxylic acid 1H-indazol-3-yl ester | 294.13 |
| 39 | 4-Methyl-piperidine-1-carboxylic acid 6-chloro-4-methyl-1H-pyrazolo[3,4-b]pyridin-3-yl ester | 309.14 |
| 40 | (3-Hydroxy-pyrazolo[3,4-b]pyridin-1-yl)-(4-methyl-piperidin-1-yl)-methanone | 261.17 |
| 41 | 4-Methyl-piperidine-1-carboxylic acid 4-methyl-3-(4-methyl-piperidine-1-carbonyloxy)-1H-pyrazolo[3,4-b]pyridin-6-yl ester | 416.24 |
| 42 | 4-Methyl-piperidine-1-carboxylic acid 6-hydroxy-4-methyl-1H-pyrazolo[3,4-b]pyridin-3- yl ester | 291.20 |
| 43 | (6-Fluoro-3-hydroxy-indazol-1-yl)-(4-methyl-piperidin-1-yl)-methanone | 278.18 |
| 44 | (6-Fluoro-3-hydroxy-indazol-1-yl)-(octahydro-isoindol-2-yl)-methanone | 304.20 |
| 45 | (3,4-Dihydro-1H-isoquinolin-2-yl)-(6-fluoro-3-hydroxy-indazol-1-yl)-methanone | 312.17 |
| 46 | Octahydro-isoindole-2-carboxylic acid 6-fluoro-1H-indazol-3-yl ester | 304.21 |
| 47 | Piperidine-1-carboxylic acid 6-fluoro-1H-indazol-3-yl ester | 264.17 |
| 48 | Methyl-phenyl-carbamic acid 6-fluoro-1H-indazol-3-yl ester | 286.17 |
| 49 | 3,4-Dihydro-1H-isoquinoline-2-carboxylic acid 6-fluoro-1H-indazol-3-yl ester | 312.13 |
| 50 | 4-Methyl-piperidine-1-carboxylic acid 1H-pyrazolo[4,3-c]pyridin-3-yl ester | 261.15 |
| 51 | 4-Methyl-piperidine-1-carboxylic acid 6-bromo-1H-indazol-3-yl ester | 338.08 |
| 52 | 3-(4-Methyl-piperidine-1-carbonyloxy)-1H-indazole-6-carboxylic acid methyl ester | 318.17 |
| 53 | (5,5,8,8-Tetramethyl-5,6,7,8-tetrahydro-naphthalen-2-yl)-carbamic acid 1H-indazol-3-yl ester | 364.8 |
| 54 | 3,4-Dihydro-1H-isoquinoline-2-carboxylic acid 6-chloro-4-methyl-1H-pyrazolo[3,4-b]pyridin-3-yl ester | 343.16 |
| 55 | 4-Methyl-piperidine-1-carboxylic acid 6-trifluoromethyl-1H-pyrazolo[3,4-b]pyridin-3-yl ester | 329.19 |
| 56 | (6-Fluoro-3-methoxy-indazol-1-yl)-(4-methyl-piperidin-1-yl)-methanone | 292.20 |
| 57 | 4-Methyl-piperidine-1-carboxylic acid 5,6-difluoro-1H-indazol-3-yl ester | 296.25 |
| 58 | (3,4-Dihydro-1H-phthalazin-2-yl)-(6-fluoro-3-hydroxy-indazol-1-yl)-methanone | 313.15 |
| 59 | (6-Chloro-3-hydroxy-4-methyl-pyrazolo[3,4-b]pyridin-1-yl)-(3,4-dihydro-1H-isoquinolin-2-yl)-methanone | 343.12 |
| 60 | 4-Methyl-piperidine-1-carboxylic acid 1-benzyl-6-hydroxy-4-methyl-1H-pyrazolo[3,4-b]pyridin-3-yl ester | 381.27 |
| 61 | 4-Methyl-piperidine-1-carboxylic acid 6-methanesulfonyl-1H-indazol-3-yl ester | 338.17 |
| 62 | 4-Methyl-piperidine-1-carboxylic acid 6-hydroxy-1H-pyrazolo[3,4-b]pyridin-3-yl ester | 277.15 |
| 63 | (3,6-Dihydroxy-pyrazolo[3,4-b]pyridin-1-yl)-(4-methyl-piperidin-1-yl)-methanone | 277.15 |
| 64 | (6-Chloro-3-hydroxy-4-methyl-pyrazolo[3,4-b]pyridin-1-yl)-(4-methyl-piperidin-1-yl)- methanone | 309.13 |
| 65 | Succinic acid methyl ester 4-methyl-3-(4-methyl-piperidine-1-carbonyloxy)-1H- pyrazolo[3,4-b]pyridin-6-yl ester | 405.26 |
| 66 | 4-Methyl-piperazine-1-carboxylic acid 6-fluoro-1H-indazol-3-yl ester; compound with trifluoro-acetic acid | 279.15 |
| 67 | 3,4-Dihydro-1H-isoquinoline-2-carboxylic acid 1H-pyrazolo[3,4-b]pyridin-3-yl ester | 295.18 |
| 68 | 4,4-Difluoro-piperidine-1-carboxylic acid 6-fluoro-1H-indazol-3-yl ester | 300.25 |
| 69 | 4,4-Difluoro-piperidine-1-carboxylic acid 4-methyl-3-(4-methyl-piperidine-1-carbonyloxy)-1H-pyrazolo[3,4- b]pyridin-6-yl ester | 438.31 |
| 70 | 6,7-Dihydro-4H-thieno[3,2-c]pyridine-5-carboxylic acid 6-fluoro-1H-indazol-3-yl ester | 318.11 |
| 71 | Piperidine-1,4-dicarboxylic acid 4-benzyl ester 1-(6-fluoro-1H-indazol-3-yl) ester | 398.26 |
| 72 | Thiomorpholine-4-carboxylic acid 6-fluoro-1H-indazol-3-yl ester | 282.13 |
| 73 | 4-Trifluoromethyl-piperidine-1-carboxylic acid 6-fluoro-1H-indazol-3-yl ester | 332.16 |
| 74 | 2,6-Dimethyl-morpholine-4-carboxylic acid 6-fluoro-1H-indazol-3-yl ester | 294.18 |
| 75 | 4-Phenyl-piperazine-1-carboxylic acid 6-fluoro-1H-indazol-3-yl ester | 341.21 |
| 76 | Morpholine-4-carboxylic acid 6-fluoro-1H-indazol-3-yl ester | 266.15 |
| 77 | 3,4-Dihydro-1H-phthalazine-2-carboxylic acid 6-fluoro-1H-indazol-3-yl ester; compound with trifluoro-acetic acid | 313.14 |
| 78 | 4,4-Difluoro-piperidine-1-carboxylic acid 6-hydroxy-4-methyl-1H-pyrazolo[3,4- b]pyridin-3-yl ester | 313.17 |
| 79 | 4,4-Difluoro-piperidine-1-carboxylic acid 6-chloro-4-methyl-1H-pyrazolo[3,4-b]pyridin-3-yl ester | 331.14 |
| 80 | 4-Methyl-piperidine-1-carboxylic acid 4,6-difluoro-1H-indazol-3-yl ester | 296.17 |
| 81 | Piperidine-1,4-dicarboxylic acid mono-(6-fluoro-1H-indazol-3-yl) ester | 308.20 |
| 82 | 4-Fluoro-piperidine-1-carboxylic acid 6-fluoro-1H-indazol-3-yl ester | 282.10 |
| 83 | 4-Cyclopropyl-piperazine-1-carboxylic acid 6-fluoro-1H-indazol-3-yl ester | 305.24 |
| 84 | Piperazine-1,4-dicarboxylic acid benzyl ester 6-fluoro-1H-indazol-3-yl ester | 399.13 |
| 85 | 4-Fluoro-piperidine-1,4-dicarboxylic acid 4-ethyl ester 1-(6-fluoro-1H-indazol-3-yl) ester | 354.13 |
| 86 | (4-Cyclopropyl-piperazin-1-yl)-(6-fluoro-3-hydroxy- | 305.35 |
| | indazol-1-yl)-methanone | |
| 87 | 4,4-Dimethyl-piperidine-1-carboxylic acid 6-fluoro-1H-indazol-3-yl ester | 292.19 |
| 88 | 4-Methyl-piperidine-1-carboxylic acid 6-fluoro-1-methyl-1H-indazol-3-yl ester | 292.13 |
| 89 | 4-(3,4-Dimethyl-phenyl)-piperazine-1-carboxylic acid 1H-indazol-3-yl ester | 351,17 |
| 90 | 4-Trifluoromethyl-piperidine-1-carboxylic acid 6-chloro-4-methyl-1H-pyrazolo[3,4-b]pyridin-3-yl ester | 263.07 |
| 91 | 4-Methyl-piperidine-1-carboxylic acid 5-fluoro-1H-indazol-3-yl ester | 278.22 |
| 92 | 4-Trifluoromethyl-piperidine-1-carboxylic acid 4-methyl-3-(4-methyl-piperidine-1-carbonyloxy)-1H-pyrazolo[3,4- b]pyridin-6-yl ester | 470.18 |
| 93 | 4-Cyclopropylidene-piperidine-1-carboxylic acid 6-fluoro-1H-indazol-3-yl ester | 302.14 |
| 94 | 4-Cyclopropyl-piperazine-1-carboxylic acid 6-hydroxy-4-methyl-1H-pyrazolo[3,4-b]pyridin-3-yl ester | 318.21 |
| 95 | Piperidine-1,4-dicarboxylic acid 4-benzyl ester 1-[4-methyl-3-(4-methyl-piperidine-1-carbonyloxy)-1H-pyrazolo[3,4-b]pyridin-6-yl] ester | 536.40 |
| 96 | 3-Methyl-piperidine-1-carboxylic acid 6-pentafluorosulfanyl 1H-indazol-3-yl ester | 386.32 |
| 97 | 4-Methyl-3-(4-methyl-piperidine-1-carbonyloxy)-1H-pyrazolo[3,4-b]pyridine-6-carboxylic acid | 319.17 |
| 98 | 4-Methyl-piperidine-1-carboxylic acid 4-methyl-6-(4-methyl-piperidine-1-carbonyl)-1H-pyrazolo[3,4-b]pyridin-3-yl ester | 400.26 |
| 99 | 4-Methyl-piperidine-1-carboxylic acid 4-methyl-6-phenyl-1H-pyrazolo[3,4-b]pyridin-3-yl ester | 351.34 |
| 100 | 4-Trifluoromethyl-piperidine-1-carboxylic acid 4,6-difluoro-1H-indazol-3-yl ester | 350.11 |
| 101 | 4-Methyl-piperidine-1-carboxylic acid 6-(4-methyl-piperidine-1-carbonyl)-1H-indazol-3-yl ester | 385.25 |
| 102 | 6-Chloro-3-hydroxy-indazole-1-carboxylic acid benzylamide | 302.10 |
| 103 | 6-Chloro-3-hydroxy-indazole-1-carboxylic acid hexylamide | 296.16 |

## Patentansprüche

1. Indazolderivat der allgemeinen Formeln I oder II, 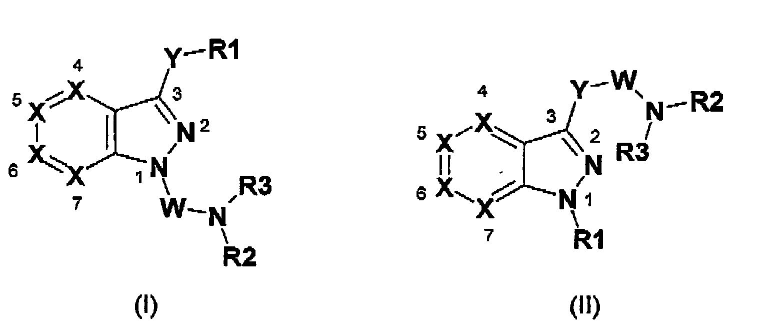 wobei bedeuten:
W -(C=O)-, -(S=O)-, -(SO₂)-;
X =C(-R)- oder =N-;
Y -O-;
R Wasserstoff, Halogen, (C₁-C₆)-Alkyl, (C₁-C₃)-Akyl-O-(C₁-C₃)-alkylen, Hydroxy, (C₁-C₆)-Alkylmercapto, Amino, (C₁-C₆)-Alkylamino, di-(C₂- C₁₂)-Alkylamino, mono-(C₁-C₆)-Alkylaminocarbonyl, di-(C₂-C₈)- Alkylaminocarbonyl, COOR4, Cyano, Trifluormethyl, (C₁-C₆)- Alkylsulfonyl, (C₁-C₆)-Alkylsulfinyl, Aminosulfonyl, Nitro, Pentafluorsulfanyl, (C₆-C₁₀)-Aryl, (C₅-C₁₂)-Heteroaryl, CO-**NR2'R3'**, O- CO-**NR2'R3',** O-CO-(C₁-C₆)-Alkylen-CO-O-(C₁-C₆)-Alkyl ,O-CO-(C₁- C₆)-Alkylen-CO-OH, O-CO-(C₁-C₆)-Alkylen-CO-**NR2'R3'** oder unsubstituiertes oder ein oder mehrfach durch F substituiertes (C₁-C₆)- Alkyloxy;
R1 H, (C₁-C₆)-Alkyl, Benzyl;
**R2'** H, (C₁-C₆)-Alkyl, (C₁-C₄)-Alkyl-Phenyl, (C₆-C₁₀)-Aryl, wobei Phenyl und Aryl ggf. durch Halogen, (C₁-C₆)-Alkyl, (C₁-C₃)-Alkyloxy, Hydroxy, (C₁- C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₆)-Alkylaminocarbonyl, (C₁- C₆)-Alkyloxycarbonyl, Cyano, Trifluormethyl, Trifluormethyloxy, (C₁-C₆)- Alkylsulfonyl, Aminosulfonyl, Nitro substituiert sein können oder Tetramethyl-tetrahydronaphthalin;
**R3'** H, (C₁-C₆)-Alkyl, oder
**R2' und R3'** zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR5-, -CR5R5-, -NR5-, -C(=O)-, -O-, -S-, -SO-, - SO₂-, ersetzt sein können, mit der Maßgabe, dass zwei Einheiten aus der Reihe -O-, -S-, -SO-, -SO₂- nicht benachbart sein dürfen;
**NR2R3** ein monocyclisches gesättigtes 5- bis 6-gliedriges Ringsystem, das in 4 Position ein Atom oder Atomglied aus der Reihe -CHR₅-, - CR5R5-, -NR5-, -O-, -S- enthält;
R4 Wasserstoff, (C₁-C₆)-Alkyl, Benzyl;
R5 (C₁-C₆)-Alkyl, Halogen, Trifluormethyl, COOR4, Cyclopropyl, Cyclopropylen;
sowie deren physiologische verträglichen Salze und tautomeren Formen.

2. Indazolderivat der Formel I oder II gemäß Anspruch 1, worin
W für -(C=O)- steht.

3. Indazolderivat der Formel I oder II gemäß den Ansprüchen 1 bis **2**, worin
X in Position 4, 5 und 7 für =C(-R)- mit R = Wasserstoff steht.

4. Indazolderivat der Formel I oder II gemäß den Ansprüchen 1 bis 3, worin
W -(C=O)-;
X =C(-R)- oder =N-;
Y -O-;
R Wasserstoff, Halogen, (C₁-C₆)-Alkyl, Hydroxy, Amino, COOR4, Trifluormethyl, (C₁-C₆)-Alkylsulfonyl, Nitro, Pentafluorsulfanyl, (C₆-C₁₀)- Aryl, CO-NR2'R3', O-CO-NR2'R3' oder O-CO-(C₁-C₆)-Alkylen-CO-O- (C₁-C₆)-Alkyl;
R1 H, (C₁-C₆)-Alkyl, Benzyl;
**R2'** (C₁-C₆)-Alkyl, Benzyl, (C₆-C₁₀)-Aryl oder Tetramethyl- tetrahydronaphthalin;
**R3'** H, (C₁-C₆)-Alkyl; oder
**R2' und R3'** zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis zwei Atome oder Atomgruppen aus der Reihe -CHR5-, -CR5R5-, -NR5-, -O-, -S-, ersetzt sein können, mit der Maßgabe, dass zwei Einheiten aus der Reihe -O-, -S- nicht benachbart sein dürfen;
**NR2R3** ein monocyclisches gesättigtes 5- bis 6-gliedriges Ringsystem, das in 4 Position ein Atom oder Atomglied aus der Reihe -CHR5-, - CR5R5-, -NR5-, -O-, -S- enthält;
R4 Wasserstoff, (C₁-C₆)-Alkyl oder Benzyl bedeuten;
R5 (C₁-C₆)-Alkyl, Halogen, Trifluormethyl, COOR4, Cyclopropyl, Cyclopropylen bedeuten.

5. Indazolderivat der Formel I gemäß den Ansprüchen 1 bis 4, worin
W -(C=O)-;
X =C(-R)- oder =N-;
Y -O-;
R Wasserstoff, Halogen, Nitro, Hydroxy oder (C₁-C₆)-Alkyl;
R1 H oder (C₁-C₆)-Alkyl;
**NR2R3** **ein monocyclisches gesättigtes 5- bis 6-gliedriges Ringsystem, das in 4 Position ein Atom oder Atomglied aus der Reihe -CHR5-, - CR5R5-, -NR5-, -O-, -S- enthält;**
R5 (C₁-C₆)-Alkyl, oder Cyclopropyl bedeuten.

6. Indazolderivat der Formel II gemäß den Ansprüchen 1 bis 5, worin
W -(C=O)-;
X =C(-R)- oder =N-:
Y -O-;
R Wasserstoff, Halogen, (C₁-C₆)-Alkyl, Hydroxy, Amino, COOR4, Trifluormethyl, (C₁-C₆)-Alkylsulfonyl, Nitro, Pentafluorsulfanyl, (C₆-C₁₀)- Aryl, CO-NR2'R3', O-CO-NR2'R3' oder O-CO-(C₁-C₆)-Alkylen-CO-O- (C₁-C₆)-Alkyl;
R1 H, (C₁-C₆)-Alkyl oder Benzyl;
**R2"** (C₁-C₆)-Alkyl, (C₆-C₁₀)-Aryl oder Tetramethyl-tetrahydronaphthalin;
**R3'** H, (C₁-C₆)-Alkyl; oder
**R2' und R3'** zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis zwei Atome oder Atomgruppen aus der Reihe -CHR5-, -CR5R5-, -NR5-, -O-, -S-, ersetzt sein können, mit der Maßgabe, dass zwei Einheiten aus der Reihe -O-, -S- nicht benachbart sein dürfen;
**NR2R3** **ein monocyclisches gesättigtes 5- bis 6-gliedriges Ringsystem, das in 4 Position ein Atom oder Atomglied aus der Reihe -CHR5-, - CR5R5-, -NR5-, -O-, -S- enthält;**
R4 Wasserstoff, (C₁-C₆)-Alkyl, Benzyl ; und
R5 (C₁-C₆)-Alkyl, Halogen, Trifluormethyl, COOR4, Cyclopropyl, Cyclopropylen bedeuten.

7. Indazolderivat der Formel II gemäß den Ansprüchen 1 bis 6, worin
NR2R3 für Piperidin steht, das in 4 Position das Atomglied CHR5 enthält.

8. Arzneimittel enthaltend ein oder mehrere der Indazolderivate der Formel I oder II gemäß einem oder mehreren der Ansprüche 1 bis 7.

9. Arzneimittel enthaltend ein oder mehrere der Indazolderivate der Formel I oder II gemäß einem oder mehreren der Ansprüche 1 bis 7 und einen oder mehrere Wirkstoffe, die günstige Wirkungen auf Stoffwechselstörungen oder damit assozierte Erkrankungen haben.

10. Arzneimittel enthaltend ein oder mehrere der Indazolderivate der Formeln I oder II, **worin**
**bedeuten:**
W -(C=O)-, -(S=O)-, -(SO₂)-;
X =C(-R)- oder =N-;
Y -O-;
R Wasserstoff, Halogen, (C₁-C₆)-Alkyl, (C₁-C₃)-Alkyl-O-(C₁-C₃)-alkylen, Hydroxy, (C₁-C₆)-Alkylmereapto, Amino, (C₁-C₆)-Alkylamino, di-(C₂- C₁₂)-Alkylamino, mono-(C₁-C₆)-Alkylaminocarbonyl, di-(C₂-C₈)- Alkylaminocarbonyl, COOR4, Cyano, Trifluormethyl, (C₁-C₆)- Alkylsulfonyl, (C₁-C₆)-Alkylsulfinyl, Aminosulfonyl, Nitro, Pentafluorsulfanyl, (C₆-C₁₀)-Aryl, (C₅-C₁₂)-Heteroaryl, CO-NR2'R3', O-CO-NR2'R3', O-CO-(C₁-C₆)-Alkylen-CO-O-(C₁-C₆)-Alkyl ,O-CO- (C₁-C₆)-Alkylen-CO-OH, O-CO-(C₁-C₆)-Alkylen-CO-NR2'R3' oder unsubstituiertes oder ein oder mehrfach durch F substituiertes (C₁-C₆)-Alkyloxy;
R1 H, (C₁-C₆)-Alkyl, Benzyl;
R2, R2' H, (C₁-C₆)-Alkyl, (C₁-C₄)-Alkyl-Phenyl, (C₆-C₁₀)-Aryl, wobei Phenyl und Aryl ggf. durch Halogen, (C₁-C₆)-Alkyl, (C₁-C₃)-Alkyloxy, Hydroxy, (C₁-C₆)-Alkylmercapto, Amino, (C₁-C₆)-Alkylamino, di-(C₂- C₁₂)-Alkylamino, mono-(C₁-C₆)-Alkylaminocarbonyl, di-(C₂-C₈)- Alkylaminocarbonyl, (C₁-C₆)-alkyloxycarbonyl, Cyano, Trifluormethyl, Trifluormethyloxy, (C₁-C₆)-Alkylsulfonyl, Aminosulfonyl, Nitro substituiert sein können oder Tetramethyl- tetrahydronaphthalin;
R3, R3' H, (C₁-C₆)-Alkyl; oder
R2 und R3 oder R2' und R3' zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR5-, -CR5R5-, -NR5-, -C(=O)-, -O-, -S-, -SO-, - SO₂-, ersetzt sein können, mit der Maßgabe, dass zwei Einheiten aus der Reihe -O-, -S-, -SO-, -SO₂- nicht benachbart sein dürfen;
R4 Wasserstoff, (C₁-C₆)-Alkyl, Benzyl;
R5 (C₁-C₆)-Alkyl, Halogen, Trifluormethyl, COOR4, Cyclopropyl, Cyclopropylen;
sowie deren physiologische verträglichen Salze und tautomeren Formen, unter der Maßgabe, dass in den Verbindungen der Formel (I) mit W = CO
a) R2 und R3 zusammen mit dem sie tragenden N-Atom ein monocyclisches oder bicyclische Ringsystem bilden, falls Y = N(R1) mit R1 = H oder (C₁-C₆)-Alkyl oder
b) YR1, R2 und R3 nicht gleichzeitig nachfolgende Bedeutungen haben können:
YR1 = OH, R2 = gegebenenfallls substituiertes (C₆-C₁₀)-Aryl und R3 = H; oder
gemäß einem oder mehreren der Ansprüche 1 bis 7 und einen oder mehrere Antidiabetika.

11. Arzneimittel mit hemmender Wirkung an der hormonsensitiven Lipase enthaltend ein oder mehrere der Indazolderivate der Formel I oder II gemäß einem oder mehreren der Ansprüche 1 bis 7 oder 10 und einen oder mehrere Lipidmodulatoren.

12. Verwendung der Indazolderivate der Formel I oder II gemäß einem oder mehreren der Ansprüche 1 bis 7 oder 10 zur Herstellung eines Medikaments zur Behandlung und/oder Prävention von Störungen des Fettsäurestoffwechsels und Glucoseverwertungsstörungen.

13. Verwendung der Indazolderivate der Formel I oder II gemäß einem oder mehreren der Ansprüche 1 bis 7 oder 10 zur Herstellung eines Medikaments zur Behandlung und/oder Prävention von Störungen, bei denen Insulin Resistenz eine Rolle spielt.

14. Verwendung der Indazolderivate der Formel I oder II gemäß einem oder mehreren der Ansprüche 1 bis 7 oder 10 zur Herstellung eines Medikaments zur Behandlung und/oder Prävention von Diabetes mellitus und der damit verbundenen Folgeerkrankungen.

15. Verwendung der Indazolderivate der Formel I oder II gemäß einem oder mehreren der Ansprüche 1 bis 7 oder 10 zur Herstellung eines Medikaments zur Behandlung und/oder Prävention von Dyslipidämien und deren Folgen.

16. Verwendung der Indazolderivate der Formel I oder II gemäß einem oder mehreren der Ansprüche 1 bis 7 oder 10 zur Herstellung eines Medikaments zur Behandlung und/oder Prävention von Zuständen, die mit dem Metabolischen Syndrom assoziert sind.

17. Verwendung der Indazolderivate der Formel I oder II gemäß einem oder mehreren der Ansprüche 1 bis 7 oder 10 in Kombination mit mindestens einem weiteren Wirkstoff zur Herstellung eines Medikaments zur Behandlung und/oder Prävention von Störungen des Fettsäurestoffwechsels und Glucoseverwertungsstörungen.

18. Verwendung der Indazolderivate der Formel I oder II gemäß einem oder mehreren der Ansprüche 1 bis 7 oder 10 in Kombination mit mindestens einem weiteren Wirkstoff zur Herstellung eines Medikaments zur Behandlung und/oder Prävention von Störungen, bei denen Insulin Resistenz eine Rolle spielt.

19. Verfahren zur Herstellung eines Arzneimittels enthaltend eine oder mehrere der Indazolderivate der Formel I oder II gemäß einem oder mehreren der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. An indazole derivative of the formulae I or II 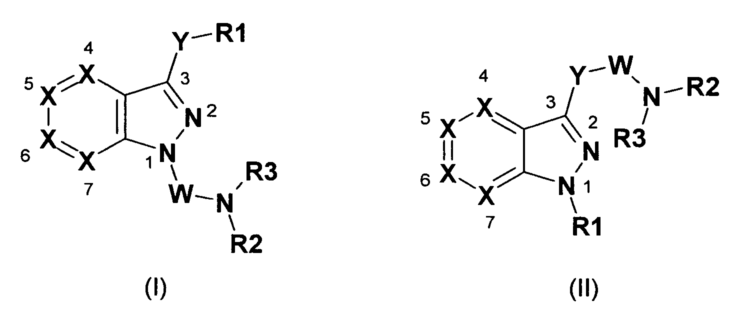 in which the meanings are:
W -(C=O)- ;
X =C(-R)- or =N-;
Y -O-;
R hydrogen, halogen, (C₁-C₆)-alkyl, (C₁-C₃)-alkyloxy-(C₁-C₃)-alkylene, hydroxy, (C₁-C₆)-alkylmercapto, amino, (C₁-C₆)-alkylamino, di-(C₂-C₁₂)- alkylamino, mono-(C₁-C₆)-alkylaminocarbonyl, di-(C₂-C₈)- alkylaminocarbonyl, COOR4, cyano, trifluoromethyl, (C₁-C₆)- alkylsulfonyl, (C₁-C₆)-alkylsulfinyl, aminosulfonyl, nitro, pentafluorosulfanyl, (C₆-C₁₀)-aryl, CO-NR2'R3', O-CO- NR2'R3', O-CO-(C₁-C₆)-alkylene-CO-O-(C₁-C₆)-alkyl, O-CO-(C₁-C₆)-alkylene- CO-OH, O-CO-(C₁-C₆)-alkylene-CO- NR2'R3'3 or unsubstituted or mono- or poly- F-substituted (C₁-C₆)-alkyloxy;
R1 H, (C₁-C₆)-alkyl, benzyl;
R2' H, (C₁-C₆)-alkyl, (C₁-C₄)-alkyl-phenyl, (C₆-C₁₀)-aryl, where phenyl or aryl may optionally be substituted by halogen, (C₁-C₆)-alkyl, (C₁-C₃)- alkyloxy, hydroxy, (C₁-C₆)-alkylmercapto, amino, (C₁-C₆)-alkylamino, di- (C₂-C₁₂)-alkylamino, mono-(C₁-C₆)-alkylaminocarbonyl, di-(C₂-C₈)- alkylaminocarbonyl, (C₁-C₆)-alkoxycarbonyl, cyano, trifluoromethyl, trifluoromethoxy, (C₁-C₆)-alkylsulfonyl, aminosulfonyl, nitro; or R2' is tetramethyl-tetrahydronaphthalene;
R3' H, (C₁-C₆)-alkyl; or R2' and R3' may form together with the nitrogen atom carrying them a saturated or partially unsaturated monocyclic, 4- to 7-membered ring system or a bicyclic saturated or partially unsaturated 8- to 14- membered ring system, whose individual members of the ring systems may be replaced by one to three atoms or atomic groups from the series -CHR5-, -CR5R5-, -NR5-, -C(=O)-, -O-, -S-, -SO-, -SO₂-, with the proviso that two units from the series -O-, -S-, -SO-, -SO₂- may not be adjacent;
NR2R3 is a monocyclic saturated 5- to 6-membered ring system which comprises in position 4 an atom or atomic member from the series -CHR5-, -CR5R5-, -NR5-, -O-, -S-;
R4 hydrogen, (C₁-C₆)-alkyl, benzyl;
R5 (C₁-C₆)-alkyl, halogen, trifluoromethyl, COOR4, cyclopropyl, cyclopropylene;
and the physiologically tolerated salts thereof as well as its tautomeric forms.

2. An indazole derivative of the formulae I or II as claimed in claim 1, in which
W is -(C=O)-.

3. An indazole derivative of the formulae I or II as claimed in claim
1 or 2, in which
X in position 4, 5 and 7 is =C(-R)- with R = hydrogen.

4. An indazole derivative of the formulae I or II as claimed in claims 1 to 3, in which
W is -(C=O)-;
X is =C(-R)- or =N-;
Y is -O-;
R is hydrogen, halogen, (C₁-C₆)-alkyl, hydroxy, amino, COOR4, trifluoromethyl, (C₁-C₆)-alkylsulfonyl, nitro, pentafluorosulfanyl, (C₆- C₁₀)-aryl, CO- NR2'R3', O-CO- NR2'R3' or O-CO-(C₁-C₆)-alkylene- CO-O-(C₁-C₆)-alkyl;
R1 is H, (C₁-C₆)-alkyl, benzyl;
R2' is (C₁-C₆)-alkyl, benzyl, (C₆-C₁₀)-aryl or tetramethyl- tetrahydronaphthalene;
R3' is H, (C₁-C₆)-alkyl; or
R2' and R3' together with the nitrogen atom carrying them may form a monocyclic saturated 5- to 6-membered ring system or a bicyclic saturated or partially unsaturated 9- to 10-membered ring system whose individual members of the ring systems may be replaced by one to two atoms or atomic groups from the series -CHR5-, -CR5R5-, -NR5-, -O-, -S-, with the proviso that two units from the series -O-, -S- may not be adjacent;
NR2R3 is a monocyclic saturated 5- to 6-membered ring system which comprises in position 4 an atom or atomic member from the series -CHR5-, -CR5R5-, -NR5-, -O-, -S-;
R4 is hydrogen, (C₁-C₆)-alkyl or benzyl;
R5 is (C₁-C₆)-alkyl, halogen, trifluoromethyl, COOR4, cyclopropyl, cyclopropylene.

5. An indazole derivative of the formula I as claimed in claims 1 to 4, in which
W is -(C=O)-;
X is =C(-R)- or =N-;
Y i s -O-;
R is hydrogen, halogen, nitro, hydroxy or (C₁-C₆)-alkyl;
R1 is H or (C₁-C₆)-alkyl;
NR2R3 is a monocyclic saturated 5- to 6-membered ring system which comprises in position 4 an atom or atomic member from the series -CHR5-, -CR5R5-, -NR5-, -O-, -S-;
R5 is (C₁-C₆)-alkyl, or cyclopropyl.

6. An indazole derivative of the formula II as claimed in claims 1 to 5, in which
W is -(C=O)-;
X is =C(-R)- or =N-;
Y is -O-;
R is hydrogen, halogen, (C₁-C₆)-alkyl, hydroxy, amino, COOR4, trifluoromethyl, (C₁-C₆)-alkylsulfonyl, nitro, pentafluorosulfanyl, (C₆-C₁₀)-aryl, CO- NR2'R3', O-CO- NR2'R3' or O-CO-(C₁-C₆)- alkylene-CO-O-(C₁-C₆)-alkyl;
R1 is H, (C₁-C₆)-alkyl or benzyl;
R2' is (C₁-C₆)-alkyl, (C₆-C₁₀)-aryl or tetramethyl-tetrahydronaphthalene;
R3' is H, (C₁-C₆)-alkyl;
R2' and R3' together with the nitrogen atom carrying them may form a monocyclic saturated 5- to 6-membered ring system or a bicyclic saturated or partially unsaturated 9- to 10-membered ring system whose individual members of the ring systems may be replaced by one to two atoms or atomic groups from the series -CHR5-, -CR5R5-, -NR5-, -O-, -S-, with the proviso that two units from the series -O-, -S- may not be adjacent;
NR2R3 is a monocyclic saturated 5- to 6-membered ring system which comprises in position 4 an atom or atomic member from the series -CHR5-, -CR5R5-, -NR5-, -O-, -S-;
R4 is hydrogen, (C₁-C₆)-alkyl, benzyl; and
R5 is (C₁-C₆)-alkyl, halogen, trifluoromethyl, COOR4, cyclopropyl, cyclopropylene.

7. An indazole derivative of the formula II as claimed in claims 1 to 6, in which
NR2R3 is piperidine which comprises the atomic member CHR5 in position 4.

8. A medicament comprising one or more of the indazole derivatives of the formula I or II as claimed in one or more of claims 1 to 7.

9. A medicament comprising one or more of the indazole derivatives of the formula I or II as claimed in one or more of claims 1 to 7, and one or more active ingredients which have favorable effects on metabolic disturbances or disorders associated therewith.

10. A medicament comprising one or more of the indazole derivatives of the formula I or II wherein the meanings are
W -(C=O)- ;
X =C(-R)- or =N-;
Y -O-;
R hydrogen, halogen, (C₁-C₆)-alkyl, (C₁-C₃)-alkyloxy-(C₁-C₃)-alkylene, hydroxy, (C₁-C₆)-alkylmercapto, amino, (C₁-C₆)-alkylamino, di-(C₂-C₁₂)- alkylamino, mono-(C₁-C₆)-alkylaminocarbonyl, di-(C₂-C₈)- alkylaminocarbonyl, COOR4, cyano, trifluoromethyl, (C₁-C₆)- alkylsulfonyl, (C₁-C₆)-alkylsulfinyl, aminosulfonyl, nitro, pentafluorosulfanyl, (C₆-C₁₀)-aryl, CO-NR2'R3', O-CO- NR2'R3', O-CO-(C₁-C₆)-alkylene-CO-O-(C₁-C₆)-alkyl, O-CO-(C₁-C₆)-alkylene- CO-OH, O-CO-(C₁-C₆)-alkylene-CO- NR2'R3'3 or unsubstituted or mono- or poly- F-substituted (C₁-C₆)-alkyloxy;
R1 H, (C₁-C₆)-alkyl, benzyl;
R2, R2' H, (C₁-C₆)-alkyl, (C₁-C₄)-alkyl-phenyl, (C₆-C₁₀)-aryl, where phenyl or aryl may optionally be substituted by halogen, (C₁-C₆)-alkyl, (C₁-C₃)- alkyloxy, hydroxy, (C₁-C₆)-alkylmercapto, amino, (C₁-C₆)-alkylamino, di- (C₂-C₁₂)-alkylamino, mono-(C₁-C₆)-alkylaminocarbonyl, di-(C₂-C₈)- alkylaminocarbonyl, (C₁-C₆)-alkoxycarbonyl, cyano, trifluoromethyl, trifluoromethoxy, (C₁-C₆)-alkylsulfonyl, aminosulfonyl, nitro; or tetramethyl-tetrahydronaphthalene;
R3, R3' H, (C₁-C₆)-alkyl; or
R2 and R3 or R2' and R3' may form together with the nitrogen atom carrying them a saturated or partially unsaturated monocyclic, 4- to 7-membered ring system or a bicyclic saturated or partially unsaturated 8- to 14- membered ring system, whose individual members of the ring systems may be replaced by one to three atoms or atomic groups from the series -CHR5-, -CR5R5-, -NR5-, -C(=O)-, -O-, -S-, -SO-, -SO₂-, with the proviso that two units from the series -O-, -S-, -SO-, -SO₂- may not be adjacent;
R4 hydrogen, (C₁-C₆)-alkyl, benzyl;
R5 (C₁-C₆)-alkyl, halogen, trifluoromethyl, COOR4, cyclopropyl, cyclopropylene;
and the physiologically tolerated salts and tautomeric forms thereof,
with the proviso that in the compounds of the formula (I) with W = CO
a) R2 and R3 form together with the nitrogen atom carrying them a monocyclic or bicyclic ring system if Y = N(R1) with R1 = H or (C₁-C₆)-alkyl or
b) Y-R1, R2 and R3 cannot simultaneously have the following meanings:
Y-R1 = OH, R2 = optionally substituted (C₆-C₁₀)-aryl and R3 = H; or
as claimed in one or more of claims 1 to 7 and one or more antidiabetics.

11. A medicament with an inhibitory effect on hormone sensitive lipase comprising one or more of the indazole derivatives of the formula I or II as claimed in one or more of claims 1 to 7 or 10 and one or more lipid modulators.

12. Use of the indazole derivatives of the formula I or II as claimed in one or more of claims 1 to 7 or 10 for the manufacture of a medicament for the treatment and/or prevention of disorders of fatty acid metabolism and glucose utilization disorders.

13. Use of the indazole derivatives of the formula I or II as claimed in one or more of claims 1 to 7 or 10 for the manufacture of a medicament for the treatment and/or prevention of disorders in which insulin resistance is involved.

14. Use of the indazole derivatives of the formula I or II as claimed in one or more of claims 1 to 7 or 10 for the manufacture of a medicament for the treatment and/or prevention of diabetes mellitus and the sequelae associated therewith.

15. Use of the indazole derivatives of the formula I or II as claimed in one or more of claims 1 to 7 or 10 for the manufacture of a medicament for the treatment and/or prevention of dyslipidemias and their sequelae.

16. Use of the indazole derivatives of the formula I or II as claimed in one or more of claims 1 to 7 or 10 for the manufacture of a medicament for the treatment and/or prevention of conditions associated with the metabolic syndrome.

17. The use of the indazole derivatives of the formula I or II as claimed in one or more of claims 1 to 7 or 10 for the manufacture of a medicament in combination with at least one further active ingredient for the treatment and/or prevention of disorders of fatty acid metabolism and glucose utilization disorders.

18. The use of the indazole derivatives of the formula I or II as claimed in one or more of claims 1 to 7 or 10 for the manufacture of a medicament in combination with at least one further active ingredient for the treatment and/or prevention of disorders in which insulin resistance is involved.

19. A process for producing a medicament comprising one or more of the indazole derivatives of the formula I or II as claimed in one or more of claims 1 to 7 or 10 for the manufacture of a medicament, which comprises mixing the active ingredient with a pharmaceutically suitable carrier, and converting this mixture into a form suitable for administration.

## Revendications

1. Dérivé d'indazole de formules I ou II 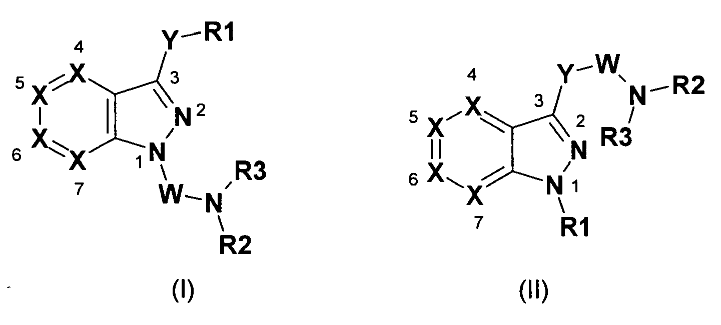 dans lesquelles les significations sont :
W -(C=O)-, -(S=O)-, -(SO₂)- ;
X =C(-R)- ou =N- ;
Y -O-;
R hydrogène, halogène, (C₁-C₆)-alkyle, (C₁-C₃)-alkyl-O-(C₁-C₃)- alkylène, hydroxy, (C₁-C₆)-alkylmercapto, amino, (C₁-C₆)- alkylamino, di-(C₂-C₁₂)-alkylamino, mono-(C₁-C₆)- alkylaminocarbonyle, di-(C₂-C₈)-alkylaminocarbonyle, COOR4, cyano, trifluorométhyle, (C₁-C₆)-alkylsulfonyle, (C₁-C₆)- alkylsulfinyle, aminosulfonyle, nitro, pentafluorosulfanyle, (C₆-C₁₀)-aryle, (C₅-C₁₂)-hétéroaryle, CO-NR2'R3', O-CO-NR2'R3', O-CO-(C₁-C₆)-alkylène-CO-O-(C₁-C₆)-alkyle, O-CO-(C₁-C₆)-alkylène-CO-OH, O-CO-(C₁-C₆)-alkylène- CO-NR2'R3' ou (C₁-C₆)-alkyloxy non substitué ou mono- ou poly- F-substitué ;
R1 H, (C₁-C₆)-alkyle, benzyle ;
R2' H, (C₁-C₆)-alkyle, (C₁-C₄)-alkylphényle, (C₆-C₁₀)-aryle, où phényle et aryle peuvent être éventuellement substitués par halogène, (C₁-C₆)-alkyle, (C₁-C₃)-alkyloxy, hydroxy, (C₁-C₆)- alkylmercapto, amino, (C₁-C₆)-alkylamino, di-(C₂-C₁₂)- alkylamino, mono-(C₁-C₆)-alkylaminocarbonyle, di-(C₂-C₈)- alkylaminocarbonyle, (C₁-C₆)-alkyloxycarbonyle, cyano, trifluorométhyle, trifluorométhyloxy, (C₁-C₆)-alkylsulfonyle, aminosulfonyle, nitro, ou tétraméthyl-tétrahydronaphtalène ;
R3' H, (C₁-C₆)-alkyle; ou
R2' et R3' peuvent former, conjointement avec l'atome d'azote qui les porte, un système de cycle monocyclique, saturé ou partiellement insaturé, à 4 à 7 chaînons ou un système de cycle bicyclique, saturé ou partiellement insaturé à 8 à 14 chaînons, les chaînons individuels des systèmes de cycles pouvant être remplacés par un à trois atomes ou groupes atomiques de la série -CHR5-, -CR5R5-, -NR5-, -C(=O)-, -O-, -S-, -SO-, -SO₂- à condition que deux unités de la série -O-, -S-, -SO-, -SO₂- ne puissent pas être adjacentes ;
NR2R3 un système de cycle monocyclique saturé à 5 à 6 chaînons, qui comprend en position 4 un atome ou élément atomique de la série -CHR5-, -CR5R5-, -NR5-, -O-, -S- ;
R4 hydrogène, (C₁-C₆)-alkyle, benzyle ;
R5 (C₁-C₆)-alkyle, halogène, trifluorométhyle, COOR4, cyclopropyle, cyclopropylène;
et les sels et formes tautomères physiologiquement tolérés de celui-ci.

2. Dérivé d'indazole de formule I ou II selon la revendication 1, dans lequel
W est -(C=O)-.

3. Dérivé d'indazole de formule I ou II selon les revendications 1 à 2, dans lequel
X en position 4, 5 et 7 est =C(-R)-, avec R = hydrogène.

4. Dérivé d'indazole de formule I ou II selon les revendications 1 à 3, dans lequel
W est -(C=O)- ;
X est =C(-R)- ou =N- ;
Y est -O- ;
R est hydrogène, halogène, (C₁-C₆)-alkyle, hydroxy, amino, COOR4, trifluorométhyle, (C₁-C₆)-alkylsulfonyle, nitro, pentafluorosulfanyle, (C₆-C₁₀)-aryle, CO-NR2'R3', O-CO- NR2'R3' ou O-CO-(C₁-C₆)-alkylène-CO-O-(C₁-C₆)-alkyle ;
R1 est H, (C₁-C₆)-alkyle, benzyle ;
R2' est (C₁-C₆)-alkyle, benzyle, (C₆-C₁₀)-aryle ou tétraméthyl- tétrahydro-naphtalène ;
R3' est H, (C₁-C₆)-alkyle ; ou
R2' et R3' conjointement avec l'atome d'azote qui les porte, peuvent former un système de cycle monocyclique saturé à 5 à 6 chaînons ou un système de cycle bicyclique saturé ou partiellement insaturé à 9 à 10 chaînons, les chaînons individuels des systèmes de cycles pouvant être remplacés par un à deux atomes ou groupes atomiques de la série -CHR5-, -CR5R5-, -NR5-, -O-, -S-, à condition que deux unités de la série -O-, -S- ne puissent pas être adjacentes ;
NR2R3 un système de cycle monocyclique saturé à 5 à 6 chaînons, qui comprend en position 4 un atome ou élément atomique de la série -CHR5-, -CR5R5-, -NR5-, -O-, -S- ;
R4 est hydrogène, (C₁-C₆)-alkyle ou benzyle ;
R5 est (C₁-C₆)-alkyle, halogène, trifluorométhyle, COOR4, cyclopropyle, cyclopropylène.

5. Dérivé d'indazole de formule I selon les revendications 1 à 4,
dans lequel
W est -(C=O)- ;
X est =C(-R)- ou =N- ;
Y est -O- ;
R est hydrogène, halogène, nitro, hydroxy ou (C₁-C₆)-alkyle ;
R1 est H ou (C₁-C₆)-alkyle ;
NR2R3 un système de cycle monocyclique saturé à 5 à 6 chaînons, qui comprend en position 4 un atome ou élément atomique de la série -CHR5-, -CR5R5-, -NR5-, -O-, -S- ;
et
R5 est (C₁-C₆)-alkyle, ou cyclopropyle.

6. Dérivé d'indazole de formule II selon les revendications 1 à 5, dans lequel
W est -(C=O)- ;
X est =C(-R)- ou =N- ;
Y est -O- ;
R est hydrogène, halogène, (C₁-C₆)-alkyle, hydroxy, amino, COOR4, trifluorométhyle, (C₁-C₆)-alkylsulfonyle, nitro, pentafluorosulfanyle, (C₆-C₁₀)-aryle, CO-NR2'R3', O-CO- NR2'R3' ou O-CO-(C₁-C₆)-alkylène-CO-O-(C₁-C₆)-alkyle ;
R1 est H, (C₁-C₆)-alkyle ou benzyle ;
R2' est (C₁-C₆)-alkyle, (C₆-C₁₀)-aryle ou tétraméthyl- tétrahydronaphtalène ;
R3' est H, (C₁-C₆)-alkyle ; ou
R2' et R3' conjointement avec l'atome d'azote qui les porte, peuvent former un système de cycle monocyclique saturé à 5 à 6 chaînons ou un système de cycle bicyclique saturé ou partiellement insaturé à 9 à 10 chaînons, les chaînons individuels des systèmes de cycles pouvant être remplacés par un à deux atomes ou groupes atomiques de la série -CHR5-, -CR5R5-, -NR5-, -O-, -S-, à condition que deux unités de la série -O-, -S- ne puissent pas être adjacentes ;
NR2R3 un système de cycle monocyclique saturé à 5 à 6 chaînons, qui comprend en position 4 un atome ou élément atomique de la série -CHR5-, -CR5R5-, -NR5-, -O-, -S- ;
R4 est hydrogène, (C₁-C₆)-alkyle, benzyle ; et
R5 est (C₁-C₆)-alkyle, halogène, trifluorométhyle, COOR4, cyclopropyle, cyclopropylène.

7. Dérivé d'indazole de formule Il selon les revendications 1 à 6, dans lequel
NR2R3 est pipéridine qui comprend l'élément atomique CHR5 en position 4.

8. Médicament comprenant un ou plusieurs des dérivés d'indazole de formule I ou II selon l'une ou plusieurs des revendications 1 à 7.

9. Médicament comprenant un ou plusieurs des dérivés d'indazole de formule I ou II selon l'une ou plusieurs des revendications 1 à 7 et un ou plusieurs principes actifs ayant des effets bénéfiques sur les perturbations métaboliques ou les troubles qui leur sont associés.

10. Médicament comprenant un ou plusieurs des dérivés d'indazole de formule I ou II dans lesquelles les significations sont :
W -(C=O)-, -(S=O)-, -(SO₂)- ;
X =C(-R)- ou =N- ;
Y -O-;
R hydrogène, halogène, (C₁-C₆)-alkyle, (C₁-C₃)-alkyl-O-(C₁-C₃)- alkylène, hydroxy, (C₁-C₆)-alkylmercapto, amino, (C₁-C₆)- alkylamino, di-(C₂-C₁₂)-alkylamino, mono-(C₁-C₆)- alkylaminocarbonyle, di-(C₂-C₈)-alkylaminocarbonyle, COOR4, cyano, trifluorométhyle, (C₁-C₆)-alkylsulfonyle, (C₁-C₆)- alkylsulfinyle, aminosulfonyle, nitro, pentafluorosulfanyle, (C₆-C₁₀)-aryle, (C₅-C₁₂)-hétéroaryle, CO-NR2'R3', O-CO-NR2'R3', O-CO-(C₁-C₆)-alkylène-CO-O-(C₁-C₆)-alkyle, O-CO-(C₁-C₆)-alkylène-CO-OH, O-CO-(C₁-C₆)-alkylène- CO-NR2'R3' ou (C₁-C₆)-alkyloxy non substitué ou mono- ou poly- F-substitué ;
R1 H, (C₁-C₆)-alkyle, benzyle ;
R2, R2` H, (C₁-C₆)-alkyle, (C₁-C₄)-alkylphényle, (C₆-C₁₀)-aryle, où phényle et aryle peuvent être éventuellement substitués par halogène, (C₁-C₆)-alkyle, (C₁-C₃)-alkyloxy, hydroxy, (C₁-C₆)- alkylmercapto, amino, (C₁-C₆)-alkylamino, di-(C₂-C₁₂)- alkylamino, mono-(C₁-C₆)-alkylaminocarbonyle, di-(C₂-C₈)- alkylaminocarbonyle, (C₁-C₆)-alkyloxycarbonyle, cyano, trifluorométhyle, trifluorométhyloxy, (C₁-C₆)-alkylsulfonyle, aminosulfonyle, nitro, ou tétraméthyl-tétrahydronaphtalène ;
R3, R3' H, (C₁-C₆)-alkyle; ou
R2 et R3 ou R2' et R3' peuvent former, conjointement avec l'atome d'azote qui les porte, un système de cycle monocyclique, saturé ou partiellement insaturé, à 4 à 7 chaînons ou un système de cycle bicyclique, saturé ou partiellement insaturé à 8 à 14 chaînons, les chaînons individuels des systèmes de cycles pouvant être remplacés par un à trois atomes ou groupes atomiques de la série -CHR5-, -NR5-, -C(=O)-, -O-, -S-, -SO-, - SO₂-, à condition que deux unités de la série -O-, -S-, -SO-, - SO₂- ne puissent pas être adjacentes ;
R4 hydrogène, (C₁-C₆)-alkyle, benzyle ;
R5 (C₁-C₆)-alkyle, halogène, trifluorométhyle, COOR4, cyclopropyle, cyclopropylène;
et les sels et formes tautomères physiologiquement tolérés de celui-ci,
à condition que dans les composés de formule (I) avec W = CO
a) R2 et R3 forment, conjointement avec l'atome d'azote qui les porte, un système de cycle monocyclique ou bicyclique si Y = N(R1), avec R1 = H ou (C₁-C₆)-alkyle ou
b) YR1, R2 et R3 ne puissent pas avoir simultanément les significations suivantes:
YR1 = OH, R2 = (C₆-C₁₀)-aryle éventuellement substitué et R3 = H
selon l'une ou plusieurs des revendications 1 à 7 et un ou plusieurs antidiabétiques.

11. Médicament ayant un effet inhibiteur sur une lipase sensible aux hormones, comprenant un ou plusieurs des dérivés d'indazole de formule I ou II selon l'une ou plusieurs des revendications 1 à 7 ou 10 et un ou plusieurs modulateurs des lipides.

12. Utilisation des dérivés d'indazole de formule I ou II selon l'une ou plusieurs des revendications 1 à 7 ou 10 pour la préparation d'un médicament pour le traitement et/ou la prévention des troubles du métabolisme des acides gras et des troubles d'utilisation du glucose.

13. Utilisation des dérivés d'indazole de formule I ou II selon l'une ou plusieurs des revendications 1 à 7 ou 10 pour la préparation d'un médicament pour le traitement et/ou la prévention de troubles dans lesquels la résistance à l'insuline est impliquée.

14. Utilisation des dérivés d'indazole de formule I ou II selon l'une ou plusieurs des revendications 1 à 7 ou 10 pour la préparation d'un médicament pour le traitement et/ou la prévention du diabète sucré et des séquelles qui lui sont associées.

15. Utilisation des dérivés d'indazole de formule I ou II selon l'une ou plusieurs des revendications 1 à 7 ou 10 pour la préparation d'un médicament pour le traitement et/ou la prévention des dyslipidémies et de leurs séquelles.

16. Utilisation des dérivés d'indazole de formule I ou II selon l'une ou plusieurs des revendications 1 à 7 ou 10 pour la préparation d'un médicament pour le traitement et/ou la prévention d'affections associées au syndrome métabolique.

17. Utilisation des dérivés d'indazole de formule I ou II selon l'une ou plusieurs des revendications 1 à 7 ou 10, en association avec au moins un principe actif supplémentaire, pour la préparation d'un médicament pour le traitement et/ou la prévention des troubles du métabolisme des acides gras et des troubles d'utilisation du glucose.

18. Utilisation des dérivés d'indazole de formule I ou II selon l'une ou plusieurs des revendications 1 à 7 ou 10, en association avec au moins un principe actif supplémentaire, pour la préparation d'un médicament pour le traitement et/ou la prévention de troubles dans lesquels la résistance à l'insuline est impliquée.

19. Procédé de production d'un médicament comprenant un ou plusieurs des dérivés d'indazole de formule I ou II selon l'une ou plusieurs des revendications 1 à 7, **caractérisé en ce que** l'on mélange le principe actif avec un support pharmaceutiquement convenable, et l'on convertit ce mélange en une forme adaptée à l'administration.
